# EUROPEAN PATENT APPLICATION

(11) **EP 4 428 229 A1**
(43) Date of publication of application: **11.09.2024**
(21) Application number: 22889998.5
(22) Date of filing: 02.11.2022
(51) Int. Cl.: C12N 5/0783, C12N 15/11

(54) **METHOD FOR PRODUCING CAR-T CELLS**

(30) Priority: 02.11.2021 JP 2021179855
(71) Applicant: AGC INC., Chiyoda-ku, Tokyo 1008405 (JP); Kyoto Prefectural Public University Corporation, Kyoto-shi, Kyoto 602-8566 (JP); Shinshu University, Matsumoto-shi, Nagano 390-8621 (JP)
(72) Inventor: KISHIMOTO, Megumi, Tokyo 100-8405 (JP); SHIMIZU, Yuto, Tokyo 100-8405 (JP); KUBOTA, Susumu, Tokyo 100-8405 (JP); YAGYU, Shigeki, Kyoto-shi, Kyoto 602-8566 (JP); SUEMATSU, Masaya, Kyoto-shi, Kyoto 602-8566 (JP); NAKAZAWA, Yozo, Matsumoto-shi, Nagano 390-8621 (JP); TANAKA, Miyuki, Matsumoto-shi, Nagano 390-8621 (JP)
(74) Representative: TBK
(86) International application number: PCT/JP2022/041076
(87) International publication number: WO 2023/080178

(57) **Abstract**

The present invention provides a method for producing a chimeric antigen receptor T (CAR-T) cell, including a step of bead separating with a specific factor a T cell from a T cell source, wherein the specific factor is CD45RA+ or the like. The method enables production of CAR-T cells high antitumor property, and can produce highly functional CAR-T cells conveniently in a short time at a low cost.

## Description

### [Technical Field]

The present invention relates to methods for producing chimeric antigen receptor T cells, particularly, methods for producing chimeric antigen receptor T cells having high antitumor property conveniently in a short time at a low cost.

### [Background Art]

As one form of immunotherapy for cancer patients, a treatment using Chimeric Antigen Receptor (CAR) T cells (hereinafter also simply referred to as CAR-T cells) has been developed in recent years, in which T cell receptor (TCR) possessed by cytotoxic T cells (CTL) is genetically modified, and CTL thus directly and selectively recognizes tumor cells and exerts an antitumor effect (Non Patent Literature 1). That is, CAR-T cell is an artificially produced T cell obtained by introducing CAR through genetic manipulation. Cancer treatment using CAR-T cells kills cancer cells through a mechanism different from that of conventional anticancer agents and radiation therapy. Therefore, it is also expected to be effective for cancers that are intractable or resistant to treatment. CAR-T cells have already been formulated for blood tumors such as some leukemias and malignant lymphomas.

In the production of CAR-T cells, autologous T cells collected from the peripheral blood of patients by apheresis etc. are used as cells into which CAR is introduced. T cells are isolated, the isolated T cells are stimulated with cytokines and antibodies, and then the CAR gene is introduced using a viral vector or transposon. For clinical use, T cells with transferred gene must be expanded and cultured to the necessary scale. Production of CAR-T cells thus requires a series of complicated production steps. Complicated production steps lead to higher drug prices. Therefore, the development of CAR-T cells having high antitumor effects that can be produced by a convenient, safe, and inexpensive method is demanded.

CAR-T cell therapy targeting CD19 has achieved remarkable success against B-cell malignant tumors; however, only half of patients with B-cell malignant tumor achieve long-term remission. Therefore, it is important to enhance the long-term functionality of CAR-T cells without affecting the antitumor effect thereof. The antitumor effect of CAR-T cells depends on various host factors, including disease state and actively hostile tumor microenvironment. Recent clinical research on CAR-T cell therapy has reported that the quality of CAR-T cell products is important for the function and antitumor effects of CAR-T cell therapy (Non Patent Literatures 2 to 4). In addition, non-viral gene transfer by genetic modification using piggybac (PB) transposon is an effective strategy for producing CAR-T cells (Non Patent Literatures 5, 6).

The properties of T cells contained in T cell sources, including peripheral blood mononuclear cells (PBMCs), influence the phenotype and function of the CAR-T cell as a final product. It has been reported that, in order to improve the production success rate and function of CAR-T cells, activation of T cells and efficiency of CAR gene introduction during the CAR-T cell production process are improved by removing monocytes and granulocytes from the starting materials and concentrating all T cells (Non Patent Literatures 7 to 9).

Patent Literatures 1 and 2 disclose a method for activating T cells into which a CAR gene has been introduced and producing CAR-T cells with high antitumor action. However, a method using an optimal subpopulation (e.g., subpopulation of PBMC) as a starting material in the process of producing CAR-T cells is not known.

Patent Literature 3 discloses a method for producing CAR-T cells by separating a fraction containing T cells by using a specific biomarker by flow cytometry, introducing a gene, and expansion culturing the obtained cells. However, a method for isolating a subpopulation, which is optimal as a starting material for the production process of CAR-T cells, by other techniques such as bead separation is not known.

Patent Literature 4 discloses a method for producing CAR-T cells by expansion culture including co-culturing T cells, into which genes have been introduced, and cells that have been engineered to express antigens. However, a method of expansion culture in the absence of feeder cells is not known.

### [Citation List]

### [Patent Literature]

[Patent Literature 1]
   WO 2021/020526
[Patent Literature 2]
   WO 2018/110374
[Patent Literature 3]
   US Patent No. 10316289
[Patent Literature 4]
   WO 2021/020526

### [Non Patent Literature]

[Non Patent Literature 1]
   Eshhar Z. et al., Proc Natl Acad Sci U S A, 1993, 90: 720-724.
[Non Patent Literature 2]
   Xu Y. et al., Blood. 2014 123(24): 3750-3759.
[Non Patent Literature 3]
   Fraietta JA. et al., Nat Med. 2018 24(5): 563-571.
[Non Patent Literature 4]
   Deng Q. et al., Nat Med. 2020 26(12): 1878-1887.
[Non Patent Literature 5]
   Kubo H. et al., Mol Ther Oncolytics. 2021 Mar 5; 20:646-658.
[Non Patent Literature 6]
   Nakamura K. et al., Mol Ther Methods Clin Dev. 2021 Mar 23; 21:315-324.
[Non Patent Literature 7]
   Noaks E. et al., Mol Ther Methods Clin Dev. 2021 20: 675-687.
[Non Patent Literature 8]
   Stroncek DF. Et al., Cytotherapy. 2016 18(7): 893-901.
[Non Patent Literature 9]
   Stroncek DF. Et al., J Transl Med. 2017 15(1): 59.

### [Summary of Invention]

### [Technical Problem]

To implement CAR-T cell therapy, CAR-T cells superior in quality (high antitumor property) and quantity (at least 1×10⁸ CAR-T cells need to be prepared) are required. However, CAR-T cells produced using conventional methods do not have sufficient antitumor property, and production of large quantities of CAR-T cells takes time, which may cause damage to the cells. Therefore, the present invention aims to provide a method that can achieve the production of CAR-T cells having high antitumor property, and a method that can achieve the production of CAR-T cells conveniently in a short time at a low cost.

### [Solution to Problem]

In view of the above-mentioned problems, the present inventors took note of cell membrane surface antigens of T cell sources, specifically peripheral blood mononuclear cells (PBMCs), that are useful as starting materials for CAR-T cell production, and investigated the relationship between them and the quality of finally-obtained CAR-T cells. As a result, it was found that more superior CAR-T cells could be obtained by using, as a starting material, a subpopulation of PBMCs having a specific expression pattern of cell membrane surface antigens. That is, CAR-T cells with higher quality can be obtained by performing a step of selecting cells (cell sorting step) by using a specific factor (which can be referred to as a cell surface marker) expressed on the cell surface, as a step before preparing T cells into which the CAR gene is introduced. Furthermore, they have found that the expansion culture of CAR-T cells can be performed in the absence of feeder cells, and that highly functional CAR-T cells can be specifically produced conveniently in a short time at a low cost by performing the expansion culture in the absence of feeder cells, and completed the present invention.

Accordingly, the present invention provides the following.
[1] A method for producing a chimeric antigen receptor T (CAR-T) cell, comprising a step of separating a T cell from a T cell source with a specific factor, wherein
   the aforementioned specific factor is at least one kind selected from the group consisting of CD3+, CD4+, CD8+, CD45RA+, CD45RO+, CD27+, CD31+, IL-7R+, CCR7+, CD62L+, CD95+, CD160+, CXCR5+, CD28+, CCL5+, CCL19+, CD45RB+, CD45RC+, CD80+, 4-1BBL+, CD3-, CD4-, CD8-, CD45RO-, CD57-, CD95-, LAG3-, CXCR3-, IL-2Rβ-, CD45RB-, and CD45RC-, and
   the aforementioned separation step is at least one kind selected from the group consisting of a density gradient separation method, an immunological cell separation technique, a magnetic cell separation technique, a nylon wool separation method, and an adhesion method.
[2] A method for producing a chimeric antigen receptor T (CAR-T) cell, comprising a step of bead separating a T cell from a T cell source with a specific factor, wherein
   the aforementioned specific factor is at least one kind selected from the group consisting of CD3+, CD4+, CD8+, CD45RA+, CD45RO+, CD27+, CD31+, IL-7R+, CCR7+, CD62L+, CD95+, CD160+, CXCR5+, CD28+, CCL5+, CCL19+, CD45RB+, CD45RC+, CD80+, 4-1BBL+, CD3-, CD4-, CD8-, CD45RO-, CD57-, CD95-, LAG3-, CXCR3-, IL-2Rβ-, CD45RB-, and CD45RC-.
[3] The production method of the above-mentioned [1] or [2], wherein the aforementioned specific factor is at least one kind selected from the group consisting of CD3+, CD4+, CD8+, CD45RA+, CD45RO+, CD27+, CD31+, IL-7R+, CCR7+, CD62L+, CD95+, CD160+, CXCR5+, CD28+, CCL5+, CCL19+, CD45RB+, CD45RC+, CD80+, and 4-1BBL+.
[4] The production method of the above-mentioned [1] or [2], wherein the aforementioned specific factor is at least one kind selected from the group consisting of CD3-, CD4-, CD8-, CD45RO-, CD57-, CD95-, LAG3-, CXCR3-, IL-2Rβ-, CD45RB-, and CD45RC-.
[5] The production method of any of the above-mentioned [1] to [4], wherein the T cell source is a peripheral blood mononuclear cell.
[6] The production method of any of the above-mentioned [2] to [5], wherein a material of the aforementioned bead is selected from the group consisting of a magnetic substance, latex, agarose, glass, cellulose, sepharose, nitrocellulose, and polystyrene.
[7] The production method of any of the above-mentioned [2] to [6], wherein the aforementioned bead has a particle size of 0.01 to 500 µm.
[8] A method for producing a chimeric antigen receptor T (CAR-T) cell, comprising a step of an activation treatment of a cell in the absence of a feeder cell.
[9] The production method of the above-mentioned [8], wherein the cell is a T cell or a T cell that expresses a chimeric antigen receptor.
[10] The production method of the above-mentioned [8] or [9], wherein the aforementioned activation treatment is performed by contacting the cell with a stimulating substance.
[11] The production method of the above-mentioned [10], wherein the aforementioned contact with the stimulating substance is contact with a substrate to which the stimulating substance is bound.
[12] The production method of the above-mentioned [11], wherein the aforementioned contact with the substrate is co-culture of the aforementioned cell and the substrate.
[13] The production method of the above-mentioned [11], wherein the aforementioned substrate is a bead or a gel.
[14] The production method of the above-mentioned [13], wherein a material of the aforementioned bead is selected from the group consisting of a magnetic substance, latex, agarose, glass, cellulose, sepharose, nitrocellulose, and polystyrene.
[15] The production method of the above-mentioned [13] or [14], wherein the aforementioned bead has a particle size of 0.01 to 500 µm.
[16] The production method of the above-mentioned [10], wherein the aforementioned contact with the stimulating substance is contact with a vesicle encapsulating the stimulating substance.
[17] The production method of the above-mentioned [16], wherein the aforementioned vesicle is a liposome, an exosome, a microvesicle, or an apoptotic body.
[18] The production method of any of the above-mentioned [8] to [10], wherein the aforementioned activation treatment is performed by culturing the aforementioned cell in a medium containing a stimulating substance.
[19] The production method of the above-mentioned [18], wherein the aforementioned activation treatment is performed by co-culturing the aforementioned cell and a substrate, to which a stimulating substance is bound, in a medium containing a stimulating substance.
[20] The production method of the above-mentioned [19], wherein the aforementioned substrate to which a stimulating substance is bound is a bead to which an antibody is bound.
[21] The method of any of the above-mentioned [10] to [20], wherein the aforementioned stimulating substance is at least one kind selected from the group consisting of a protein, a peptide fragment, and a glycan.
[22] The production method of any of the above-mentioned [10] to [21], wherein the aforementioned stimulating substance is at least one kind selected from the group consisting of CD3+, CD4+, CD8+, CD45RA+, CD45RO+, CD27+, CD31+, IL-7R+, CCR7+, CD62L+, CD95+, CD160+, CXCR5+, CD28+, CCL5+, CCL19+, CD45RB+, CD45RC+, CD80+, 4-1BBL+, CD3-, CD4-, CD8-, CD45RO-, CD57-, CD95-, LAG3-, CXCR3-, IL-2Rβ-, BCL2L14-, PPAR-, statin-, CD45RB-, and CD45RC-.
[23] A method for producing a chimeric antigen receptor T (CAR-T) cell, comprising a step of an antigen recognition treatment of a cell in the absence of a feeder cell.
[24] The production method of the above-mentioned [23], wherein the cell is a T cell or a T cell that expresses a chimeric antigen receptor.
[25] The production method of the above-mentioned [23] or [24], wherein the aforementioned antigen recognition treatment is performed by contacting the cell with a recognition substance.
[26] The production method of the above-mentioned [25], wherein the aforementioned contact with the recognition substance is contact with a substrate to which a recognition substance is bound.
[27] The production method of the above-mentioned [26], wherein the aforementioned contact with the substrate is co-culture of the aforementioned cell and the substrate.
[28] The production method of the above-mentioned [26], wherein the aforementioned substrate is a bead or a gel.
[29] The production method of the above-mentioned [28], wherein a material of the aforementioned bead is selected from the group consisting of a magnetic substance, latex, agarose, glass, cellulose, sepharose, nitrocellulose, and polystyrene.
[30] The production method of the above-mentioned [28] or [29], wherein the aforementioned bead has a particle size of 0.01 to 500 µm.
[31] The production method of the above-mentioned [25], wherein the aforementioned contact with the recognition substance is contact with a vesicle encapsulating a recognition substance.
[32] The production method of the above-mentioned [31], wherein the aforementioned vesicle is a liposome, an exosome, a microvesicle, or an apoptotic body.
[33] The production method of any of the above-mentioned [23] to [25], wherein the aforementioned antigen recognition treatment is performed by culturing the aforementioned cell in a medium containing a recognition substance.
[34] The production method of the above-mentioned [33], wherein the aforementioned antigen recognition treatment is performed by co-culturing the aforementioned cell and a substrate, to which a recognition substance is bound, in a medium containing a recognition substance.
[35] The production method of the above-mentioned [34], wherein the aforementioned substrate to which a recognition substance is bound is a bead to which an antibody is bound.
[36] The production method of any of the above-mentioned [25] to [35], wherein the aforementioned recognition substance is at least one kind selected from the group consisting of a protein, a peptide fragment, and a glycan.
[37] The production method of any of the above-mentioned [25] to [36], wherein the aforementioned recognition substance is at least one kind selected from the group consisting of EPHB4, HER2, and CD19.
[38] The production method of any of the above-mentioned [8] to [37], wherein a cell after CAR gene introduction and a bead, to which a stimulating substance and a recognition substance are bound, are co-cultured in a medium containing a stimulating substance and a recognition substance, wherein
   the aforementioned stimulating substance is at least one kind selected from the group consisting of CD3+, CD4+, CD8+, CD45RA+, CD45RO+, CD27+, CD31+, IL-7R+, CCR7+, CD62L+, CD95+, CD160+, CXCR5+, CD28+, CCL5+, CCL19+, CD45RB+, CD45RC+, CD80+, 4-1BBL+, CD3-, CD4-, CD8-, CD45RO-, CD57-, CD95-, LAG3-, CXCR3-, IL-2Rβ-, BCL2L14-, PPAR-, statin-, CD45RB-, and CD45RC-, and
   the aforementioned recognition substance is at least one kind selected from the group consisting of EPHB4, HER2, and CD19.
[39] A method for producing a chimeric antigen receptor T (CAR-T) cell, comprising
   a step of separating the T cell from a T cell source with a specific factor,
   a step of introducing a CAR gene into the separated cell, and
   a step of expansion culture of the cell into which the CAR gene has been introduced, wherein
   the aforementioned specific factor is at least one kind selected from the group consisting of CD3+, CD4+, CD8+, CD45RA+, CD45RO+, CD27+, CD31+, IL-7R+, CCR7+, CD62L+, CD95+, CD160+, CXCR5+, CD28+, CCL5+, CCL19+, CD45RB+, CD45RC+, CD80+, 4-1BBL+, CD3-, CD4-, CD8-, CD45RO-, CD57-, CD95-, LAG3-, CXCR3-, IL-2Rβ-, CD45RB-, and CD45RC-.
[40] The production method of the above-mentioned [39], wherein the aforementioned separation step is at least one kind selected from the group consisting of a density gradient separation method, an immunological cell separation technique, a magnetic cell separation technique, a nylon wool separation method, and an adhesion method.
[41] The production method of the above-mentioned [39], wherein the aforementioned separation step is bead separation.
[42] The production method of any of the above-mentioned [39] to [41], wherein the aforementioned specific factor is at least one kind selected from the group consisting of CD3+, CD4+, CD8+, CD45RA+, CD45RO+, CD27+, CD31+, IL-7R+, CCR7+, CD62L+, CD95+, CD160+, CXCR5+, CD28+, CCL5+, CCL19+, CD45RB+, CD45RC+, CD80+, and 4-1BBL+.
[43] The production method of any of the above-mentioned [39] to [41], wherein the aforementioned specific factor is at least one kind selected from the group consisting of CD3-, CD4-, CD8-, CD45RO-, CD57-, CD95-, LAG3-, CXCR3-, IL-2Rβ-, CD45RB-, and CD45RC-.
[44] The production method of any of the above-mentioned [39] to [43], wherein the T cell source is a peripheral blood mononuclear cell.
[45] The production method of any of the above-mentioned [41] to [44], wherein a material of the aforementioned bead is selected from the group consisting of a magnetic substance, latex, agarose, glass, cellulose, sepharose, nitrocellulose, and polystyrene.
[46] The production method of any of the above-mentioned [41] to [45], wherein the aforementioned bead has a particle size of 0.01 to 500 µm.
[47] The production method of the above-mentioned [39], wherein the aforementioned expansion culture comprises an activation treatment and an antigen recognition treatment.
[48] The production method of the above-mentioned [47], wherein the aforementioned activation treatment is performed by contacting the cell with a stimulating substance.
[49] The production method of the above-mentioned [48], wherein the aforementioned contact with the stimulating substance is contact with a substrate to which the stimulating substance is bound.
[50] The production method of the above-mentioned [49], wherein the aforementioned contact with the substrate is co-culture of the aforementioned cell and the substrate.
[51] The production method of the above-mentioned [48] or [49], wherein the aforementioned substrate is a bead or a gel.
[52] The production method of the above-mentioned [51], wherein a material of the aforementioned bead is selected from the group consisting of a magnetic substance, latex, agarose, glass, cellulose, sepharose, nitrocellulose, and polystyrene.
[53] The production method of the above-mentioned [51] or [52], wherein the aforementioned bead has a particle size of 0.01 to 500 µm.
[54] The production method of the above-mentioned [48], wherein the aforementioned contact with the stimulating substance is contact with a vesicle encapsulating the stimulating substance.
[55] The production method of the above-mentioned [54], wherein the aforementioned vesicle is a liposome, an exosome, a microvesicle, or an apoptotic body.
[56] The production method of the above-mentioned [47] or [48], wherein the aforementioned activation treatment is performed by culturing the aforementioned cell in a medium containing a stimulating substance.
[57] The production method of the above-mentioned [56], wherein the aforementioned activation treatment is performed by co-culturing the aforementioned cell and a substrate, to which a stimulating substance is bound, in a medium containing a stimulating substance.
[58] The production method of the above-mentioned [57], wherein the aforementioned substrate to which a stimulating substance is bound is a bead to which an antibody is bound.
[59] The production method of any of the above-mentioned [48] to [58], wherein the aforementioned stimulating substance is at least one kind selected from the group consisting of a protein, a peptide fragment, and a glycan.
[60] The production method of any of the above-mentioned [48] to [59], wherein the aforementioned stimulating substance is at least one kind selected from the group consisting of CD3+, CD4+, CD8+, CD45RA+, CD45RO+, CD27+, CD31+, IL-7R+, CCR7+, CD62L+, CD95+, CD160+, CXCR5+, CD28+, CCL5+, CCL19+, CD45RB+, CD45RC+, CD80+, 4-1BBL+, CD3-, CD4-, CD8-, CD45RO-, CD57-, CD95-, LAG3-, CXCR3-, IL-2Rβ-, BCL2L14-, PPAR-, statin-, CD45RB-, and CD45RC-.
[61] The production method of the above-mentioned [47], wherein the aforementioned antigen recognition treatment is performed by contacting the cell with a recognition substance.
[62] The production method of the above-mentioned [61], wherein the aforementioned contact with the recognition substance is contact with a substrate to which a recognition substance is bound.
[63] The production method of the above-mentioned [62], wherein the aforementioned contact with the substrate is co-culture of the aforementioned cell and the substrate.
[64] The production method of the above-mentioned [62] or [63], wherein the aforementioned substrate is a bead or a gel.
[65] The production method of the above-mentioned [64], wherein a material of the aforementioned bead is selected from the group consisting of a magnetic substance, latex, agarose, glass, cellulose, sepharose, nitrocellulose, and polystyrene.
[66] The production method of the above-mentioned [64] or [65], wherein the aforementioned bead has a particle size of 0.01 to 500 µm.
[67] The production method of the above-mentioned [61], wherein the aforementioned contact with the recognition substance is contact with a vesicle encapsulating the recognition substance.
[68] The production method of the above-mentioned [67], wherein the aforementioned vesicle is a liposome, an exosome, a microvesicle, or an apoptotic body.
[69] The production method of the above-mentioned [47] or [61], wherein the aforementioned antigen recognition treatment is performed by culturing the aforementioned cell in a medium containing a recognition substance.
[70] The production method of the above-mentioned [69], wherein the aforementioned antigen recognition treatment is performed by co-culturing the aforementioned cell and a substrate, to which a recognition substance is bound, in a medium containing a recognition substance.
[71] The production method of the above-mentioned [70], wherein the aforementioned substrate to which a recognition substance is bound is a bead to which an antibody is bound.
[72] The production method of any of the above-mentioned [61] to [71], wherein the aforementioned recognition substance is at least one kind selected from the group consisting of a protein, a peptide fragment, and a glycan.
[73] The production method of the above-mentioned [72], wherein the aforementioned recognition substance is at least one kind selected from the group consisting of EPHB4, HER2, and CD19.
[74] The production method of any of the above-mentioned [47] to [73], wherein a cell after CAR gene introduction and a bead, to which a stimulating substance and a recognition substance are bound, are co-cultured in a medium containing a stimulating substance and a recognition substance, wherein

The aforementioned stimulating substance is at least one kind selected from the group consisting of CD3+, CD4+, CD8+, CD45RA+, CD45RO+, CD27+, CD31+, IL-7R+, CCR7+, CD62L+, CD95+, CD160+, CXCR5+, CD28+, CCL5+, CCL19+, CD45RB+, CD45RC+, CD80+, 4-1BBL+, CD3-, CD4-, CD8-, CD45RO-, CD57-, CD95-, LAG3-, CXCR3-, IL-2Rβ-, BCL2L14-, PPAR-, statin-, CD45RB-, and CD45RC-, and
the aforementioned recognition substance is at least one kind selected from the group consisting of EPHB4, HER2, and CD19.

In another embodiment, the present invention provides the following.
[1'] A method for producing a chimeric antigen receptor T (CAR-T) cell, comprising a step of separating the T cell from a T cell source with a specific factor, wherein
   the aforementioned specific factor is at least one kind selected from the group consisting of CD3+, CD4+, CD8+, CD45RA+, CD27+, IL-7R+, CCR7+, CD62L+, CD95+, CXCR5+, CD28+, CCL5+, CCL19+, CD45RB+, CD45RC+, CD80+, 4-1BBL+, CD44+, CD45RO-, CD57-LAG3-, CXCR3-, and IL-2Rβ-, and
   the aforementioned separation step is at least one kind selected from the group consisting of a density gradient separation method, an immunological cell separation technique, a magnetic cell separation technique, a nylon wool separation method, and an adhesion method.
[2'] A method for producing a chimeric antigen receptor T (CAR-T) cell, comprising a step of bead separating a T cell from a T cell source with a specific factor, wherein
   the aforementioned specific factor is at least one kind selected from the group consisting of CD3+, CD4+, CD8+, CD45RA+, CD27+, IL-7R+, CCR7+, CD62L+, CD95+, CXCR5+, CD28+, CCL5+, CCL19+, CD45RB+, CD45RC+, CD80+, 4-1BBL+, CD44+, CD45RO-, CD57-, LAG3-, CXCR3-, and IL-2Rβ-.
[3'] The production method of the above-mentioned [1'] or [2'], wherein the aforementioned specific factor is at least one kind selected from the group consisting of CD3+, CD45RA+, IL-7R+, CD62L+, and CD28+.
[4'] The production method of any of the above-mentioned [1'] to [3'], wherein the T cell source is a peripheral blood mononuclear cell.
[5'] A method for producing a chimeric antigen receptor T (CAR-T) cell, comprising a step of an activation treatment of a cell in the absence of a feeder cell.
[6'] The production method of [5'], wherein the cell is a T cell or a T cell that expresses a chimeric antigen receptor.
[7'] The production method of [5'] or [6'], wherein the aforementioned activation treatment is performed by contacting the cell with a stimulating substance.
[0012] [8'] The production method of [7'], wherein the aforementioned contact with the stimulating substance is contact with a substrate to which the stimulating substance is bound.
[9'] The production method of [8'], wherein the aforementioned contact with the substrate is co-culture of the aforementioned cell and the substrate.
[10'] The production method of any of [5'] to [7'], wherein the aforementioned activation treatment is performed by culturing the aforementioned cell in a medium containing a stimulating substance.
[11'] The production method of [10'], wherein the aforementioned activation treatment is performed by co-culturing the aforementioned cell and a substrate, to which a stimulating substance is bound, in a medium containing a stimulating substance.
[12'] The production method of [11'], wherein the aforementioned substrate to which a stimulating substance is bound is a bead to which a protein is bound.
[13'] The production method of any of [7'] to [12'], wherein the aforementioned stimulating substance is at least one kind selected from the group consisting of CD4+, CD8+, CD45RA+, CCR7+, CD62L+, CXCR5+, CCL5+, CD327+, CD73+, CCL19+, CD45RB+, CD3-, CD45RO-, CD57-, LAG3-, CXCR3-, IL-2Rβ-, BCL2L14-, MEK1-, MEK2-, mTOR-, PPAR-, and statin-.
[14'] The production method of [13'], wherein the aforementioned stimulating substance is at least one kind selected from the group consisting of CCR7+, CD62L+, CXCR5+, CCL5+, CD327+, CD73+, SB431542-, PD0325901-, Rapamycin-, simvastatin-, GW9662-, Rosiglitazone-, and GW6471-.
[15'] A method for producing a chimeric antigen receptor T (CAR-T) cell, comprising a step of an antigen recognition treatment of a cell in the absence of a feeder cell.
[16'] The production method of [15'], wherein the cell is a T cell or a T cell that expresses a chimeric antigen receptor.
[17'] The production method of [15'] or [16'], wherein the aforementioned antigen recognition treatment is performed by contacting the cell with a recognition substance.
[18'] The production method of [17'], wherein the aforementioned contact with the recognition factor is contact with a substrate to which a recognition substance is bound.
[19'] The production method of [18'], wherein the aforementioned contact with the substrate is co-culture of the aforementioned cell and the substrate.
[20'] The production method of any of [15'] to [17'], wherein the aforementioned antigen recognition treatment is performed by culturing the aforementioned cell in a medium containing a recognition substance.
[21'] The production method of [20'], wherein the aforementioned antigen recognition treatment is performed by co-culturing the aforementioned cell and a substrate, to which a recognition substance is bound, in a medium containing a recognition substance.
[22'] The production method of [21'], wherein the aforementioned substrate to which the recognition substance is bound is a bead to which a protein of the recognition substance is bound.
[23'] The production method of any of [17'] to [22'], wherein the aforementioned recognition substance is at least one kind selected from the group consisting of EPHB4, HER2, and CD19.
[24'] The production method of any of [5'] to [23'], wherein a cell after CAR gene introduction and a bead, to which a stimulating substance and a recognition substance are bound, are co-cultured in a medium containing a stimulating substance and a recognition substance, wherein
   the aforementioned stimulating substance is at least one kind selected from the group consisting of CCR7+, CD62L+, CXCR5+, CCL5+, CD327+, CD73+, SB431542-, PD0325901-, Rapamycin-, simvastatin-, GW9662-, Rosiglitazone-, and GW6471-, and
   the aforementioned recognition substance is at least one kind selected from the group consisting of EPHB4, HER2, and CD19.
[25'] A method for producing a chimeric antigen receptor T (CAR-T) cell, comprising
   a step of separating a T cell from a T cell source with a specific factor,
   a step of introducing a CAR gene into the separated cell, and
   a step of expansion culture of the cell into which the CAR gene has been introduced, wherein
   the aforementioned specific factor is at least one kind selected from the group consisting of CD3+, CD45RA+, CD27+, IL-7R+, CD62L+, CD28+, CD44+, CD45RO-, and CD57-.
[26'] The production method of [25'], wherein the aforementioned separation step is bead separation.
[27'] The production method of [25'] or [26'], wherein the aforementioned specific factor is at least one kind selected from the group consisting of CD3+, CD45RA+, IL-7R+, CD62L+, and CD28+.
[28'] The production method of [25'], wherein the aforementioned expansion culture comprises an activation treatment and an antigen recognition treatment.
[29'] The production method of [28'], wherein the aforementioned activation treatment is performed by contacting the cell with a stimulating substance.
[30'] The production method of [29'], wherein the aforementioned contact with the stimulating substance is contact with a substrate to which the stimulating substance is bound.
[31'] The production method of [30'], wherein the aforementioned contact with the substrate is co-culture of the aforementioned cell and the substrate.
[32'] The production method of [28'] or [29'], wherein the aforementioned activation treatment is performed by culturing the aforementioned cell in a medium containing a stimulating substance.
[33'] The production method of [32'], wherein the aforementioned activation treatment is performed by co-culturing the aforementioned cell and a substrate, to which a stimulating substance is bound, in a medium containing a stimulating substance.
[34'] The production method of [33'], wherein the aforementioned substrate to which a stimulating substance is bound is a bead to which a protein is bound.
[35'] The production method of any of [29'] to [34'], wherein the aforementioned stimulating substance is at least one kind selected from the group consisting of CCR7+, CD62L+, CXCR5+, CCL5+, CD327+, CD73+, SB431542-, PD0325901-, Rapamycin-, simvastatin-, GW9662-, Rosiglitazone-, and GW6471-.
[36'] The production method of [28'], wherein the aforementioned antigen recognition treatment is performed by contacting the cell with a recognition substance.
[37'] The production method of [36'], wherein the aforementioned contact with the recognition substance is contact with a substrate to which a recognition substance is bound.
[38'] The production method of [37'], wherein the aforementioned contact with the substrate is co-culture of the aforementioned cell and the substrate.
[39'] The production method of [28'] or [36'], wherein the aforementioned antigen recognition treatment is performed by culturing the aforementioned cell in a medium containing a recognition substance.
[40'] The production method of [39'], wherein the aforementioned antigen recognition treatment is performed by co-culturing the aforementioned cell and a substrate, to which a recognition substance is bound, in a medium containing a recognition substance.
[41'] The production method of [40'], wherein the aforementioned substrate to which a recognition substance is bound is a bead to which a protein is bound.
[42'] The production method of [39'], wherein the aforementioned recognition substance is at least one kind selected from the group consisting of EPHB4, HER2, and CD19.
[43'] The production method of any of [28'] to [42'], wherein a cell after CAR gene introduction and a bead, to which a stimulating substance and a recognition substance are bound, are co-cultured in a medium containing a stimulating substance and a recognition substance, wherein
   the aforementioned stimulating substance is at least one kind selected from the group consisting of CCR7+, CD62L+, CXCR5+, CCL5+, CD327+, CD73+, SB431542-, PD0325901-, Rapamycin-, simvastatin-, GW9662-, Rosiglitazone-, and GW6471-, and
   the aforementioned recognition substance is at least one kind selected from the group consisting of EPHB4, HER2, and CD19.
[44'] A method for producing a chimeric antigen receptor T (CAR-T) cell, comprising co-culturing, in the absence of a feeder cell and in a medium containing a stimulating substance, a cell after CAR gene introduction and a bead, to which a co-stimulating substance and a recognition substance are bound, wherein
   the aforementioned stimulating substance is at least one kind selected from the group consisting of an anti-CCR7 antibody, an anti-CD62L antibody, an anti-CXCR5 antibody, an anti-CCL5 antibody, an anti-CD327 antibody, an anti-CD73 antibody, SB431542, PD0325901, Rapamycin, simvastatin, GW9662, Rosiglitazone, and GW6471,
   the aforementioned recognition substance is at least one kind selected from the group consisting of EPHB4, HER2, and CD19,
   the aforementioned co-stimulating substance is at least one kind selected from the group consisting of CD80, CD86, 4-1BBL, OX40L, ICOS-L, CD70, CD40L, CD270, ICAM-1, LFA-3CD72, CD55, VCAM-1, MadCAM-1, CD111, CD112, CD155, CD153, PD-L2, PD-L1, Galectin-9, MHC, and CD113.
[45'] The production method of [44'], wherein the cell after CAR gene introduction is a cell derived from a T cell separated with a specific factor from a T cell source, and
   the aforementioned specific factor is at least one kind selected from the group consisting of CD3+, CD45RA+, IL-7R+, CD62L+, and CD28+.

### [Advantageous Effects of Invention]

According to the method of the present invention, CAR-T cells with higher quality can be obtained, and highly functional CAR-T cells can be produced conveniently in a short time at a low cost.

### [Brief Description of Drawings]

[Fig. 1]

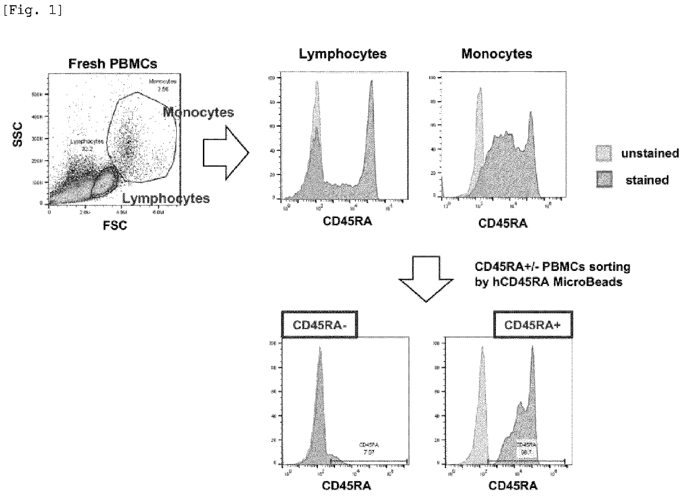

   Fig. 1 is a diagram showing the isolation process of CD45RA+ PBMC and CD45RA- PBMC from total PBMC.
[Fig. 2]

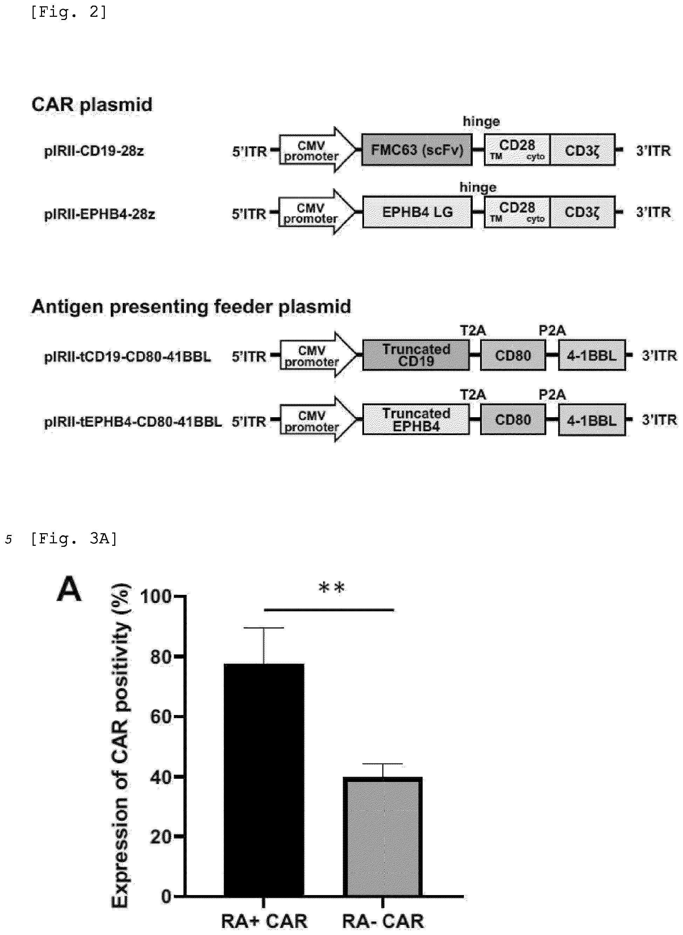

   Fig. 2 presents diagrams of the construction of transposon plasmids for CAR-T cells and antigen-presenting feeder cells. ITR; Internal Tandem Repeat, TM; transmembrane domain, cyto; cytoplasmic domain.
[Fig. 3A]
   Fig. 3A is a graph showing the results of examining the expression of the CAR transgene 24 hours after introduction into CD45RA+ cells (RA+) or CD45RA- cells (RA-) (n=3, 3 donors). It is expressed as the percentage (%) of cells expressing the CAR transgene. ** P <0.01.
[Fig. 3B]

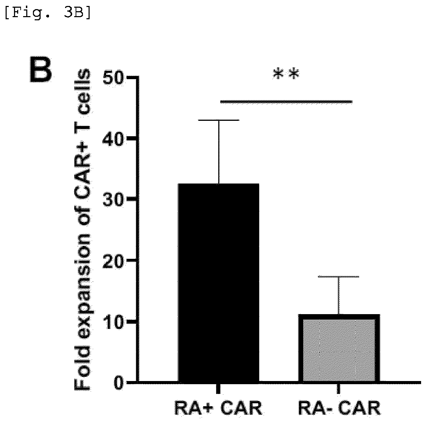

   Fig. 3B is a graph showing the results of examining the degree of proliferation of CAR-positive T cells 14 days after introduction into CD45RA+ cells (RA+) or CD45RA- cells (RA-) (n=5, 3 donors). The degree of proliferation of CAR-positive T cells is expressed as fold.
[Fig. 3C]

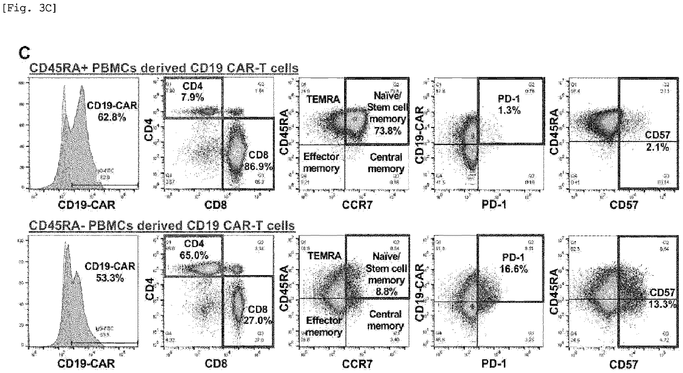

   Fig. 3C shows the results of examining the representative expression and phenotype of CD19 CAR-T cells derived from CD45RA+ cells or CD19 CAR-T cells derived from CD45RA- cells, and the result of examining the expression of exhaustion markers by flow cytometry in both CAR-T cells.
[Fig. 3D]

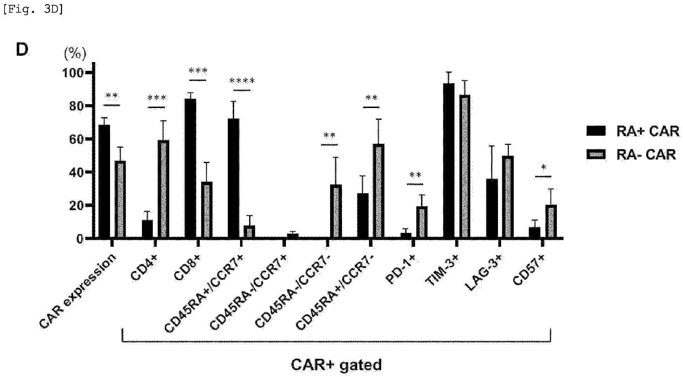

   Fig. 3D is a diagram showing the phenotype and expression status of exhaustion markers in CD19 CAR-T cells derived from CD45RA+ cells (RA+) or CD19 CAR-T cells derived from CD45RA-cells (RA-) (n=3-6, 3 donors). All data are shown as mean±standard deviation. * P <0.05, ** P <0.01, *** P <0.001, **** P <0.0001.
[Fig. 4A]

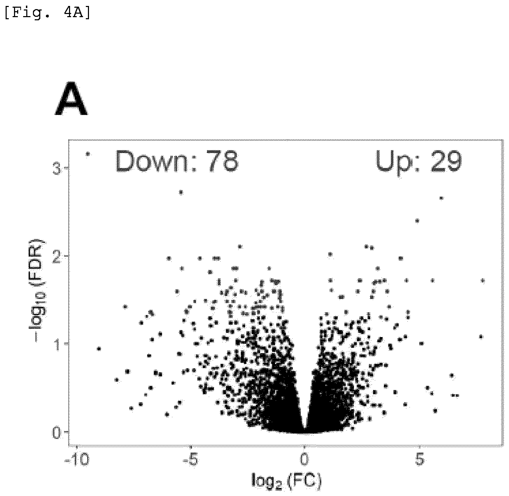

   Fig. 4A is a Volcano plot showing genes that show log2 fold change>0.9 (29 genes) or <-0.9 (78 genes) and FDR after adjustment <0.05 in CD45RA+ CAR-T as compared with CD45RA- CAR-T and that show different expression.
[Fig. 4B]

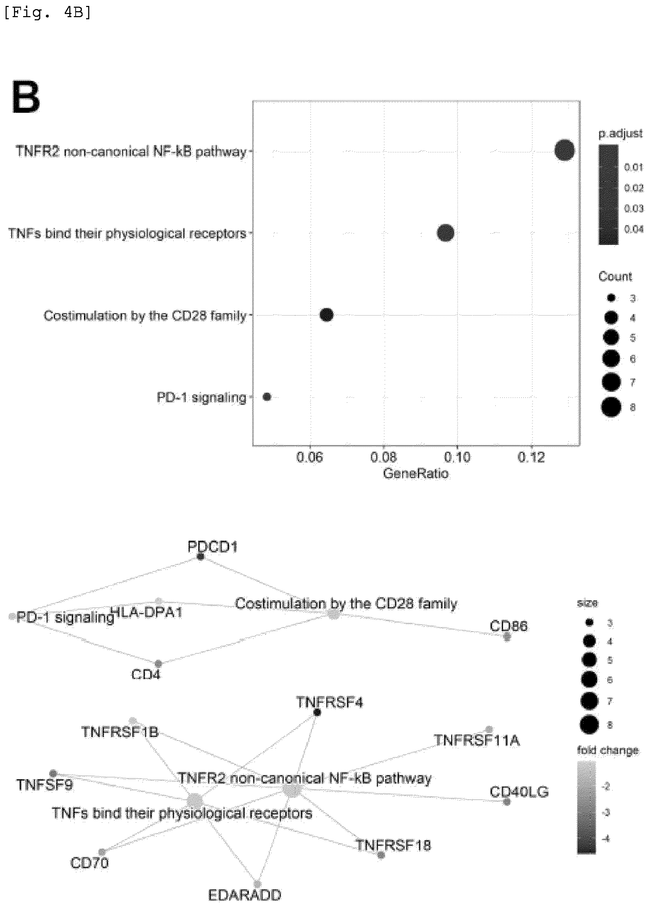

   Fig. 4B presents diagrams showing the results of reactome pathway analysis. Several gene pathways were significantly downregulated in CD45RA+ CAR-T cells as compared with CD45RA-CAR-T.
[Fig. 5A]

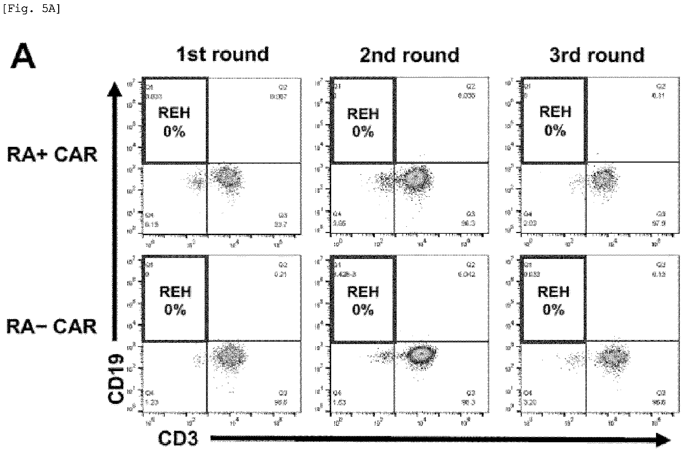

   Fig. 5A is a diagram showing the analysis results of the proportions of each cell when CAR-T cells and REH cells were continuously co-cultured, by continuous in vitro tumor challenge assay. Representative dot plots are shown.
[Fig. 5B]

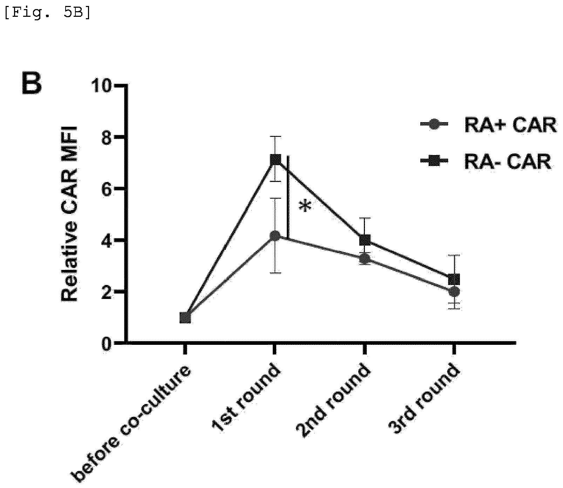

   Fig. 5B is a diagram showing the analysis results of CAR function by continuous in vitro tumor challenge assay. Relative CAR mean fluorescence intensity (MFI) of CAR-T cells during continuous co-culture (MFI=1, before co-culture) (n=3). * P <0.05.
[Fig. 5C]

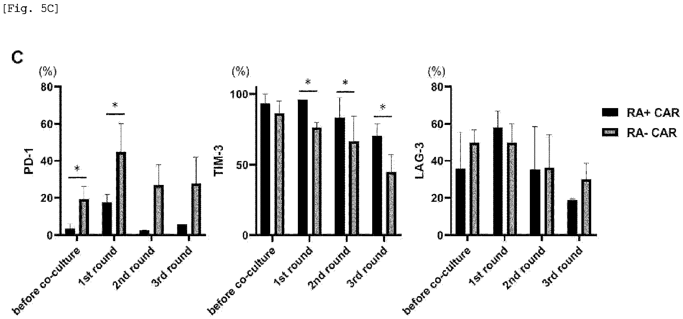

   Fig. 5C presents diagrams showing the analysis results of CAR function by continuous in vitro tumor challenge assays. The results of examining the expression of PD-1, TIM-3, and LAG-3 in CAR-T cells during continuous co-culture are shown (n=3). All data are shown as mean±standard deviation. * P<0.05. PD-1: programmed cell death protein-1, TIM-3: T cell immunoglobulin mucin-3, LAG-3: Lymphocyte-activation gene 3
[Fig. 6A]

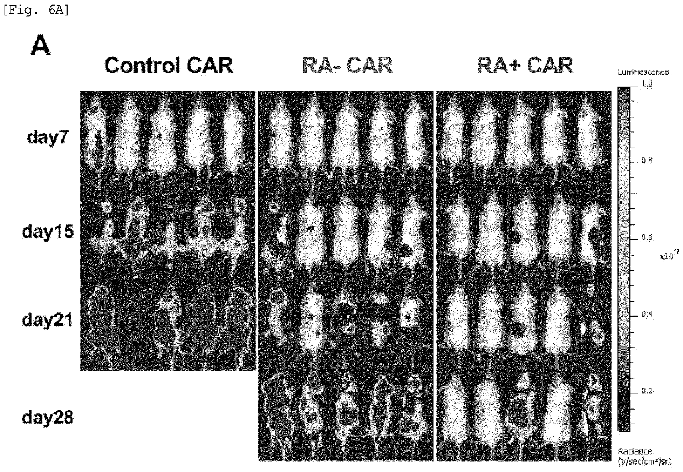

   Fig. 6A is a diagram showing bioluminescence images of 5 NSG mice/group after each CAR-T cell was intravenously injected.
[Fig. 6B]

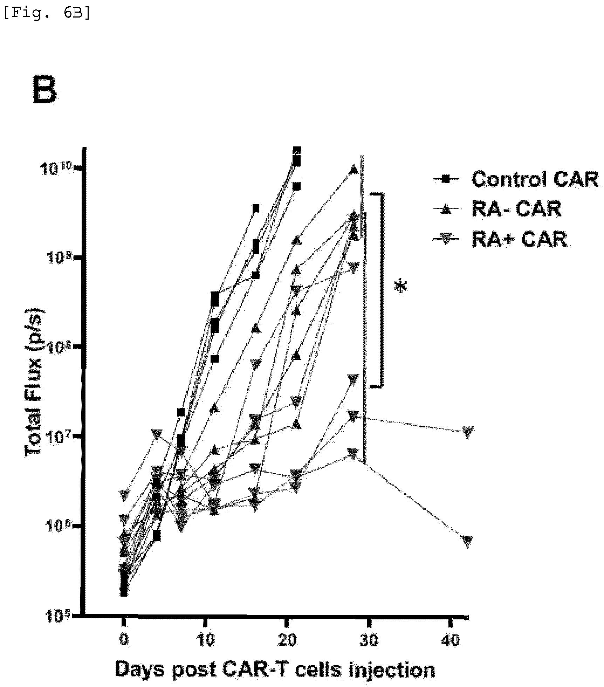

   Fig. 6B shows the tumor volume of each mouse measured as total flux (p/s). The CD45RA+ CAR-T group showed statistically significant tumor reduction, which is measured as mean total flux at day 28, as compared with the CD45RA- CAR-T group. * P <0.05.
[Fig. 6C]

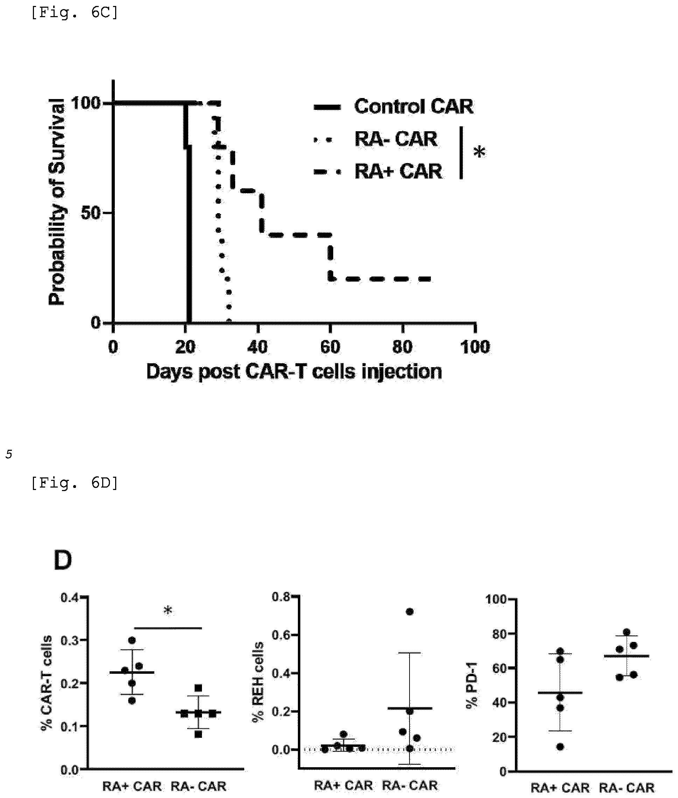

   Fig. 6C shows a Kaplan-Meier plot of overall survival rate (each group, n=5). The CD45RA+ CAR-T group achieved prolonged tumor control as compared with the CD45RA- CAR-T group. Log-rank test. * P <0.05.
[Fig. 6D]
   Fig. 6D presents diagrams showing the results of bone marrow analysis 15 days after CAR-T cell injection. Flow cytometry confirmed CAR-T cells (left), REH cells (middle), and PD-1 expression on CAR-T cells (right). * P <0.05.
[Fig. 6E]
   CAR-T cells and REH cells in the bone marrow of mice on days 15, 25, and 50 in long-lived mice injected with RA+ CAR-T cells. Representative dot plot data is shown.
[Fig. 7]

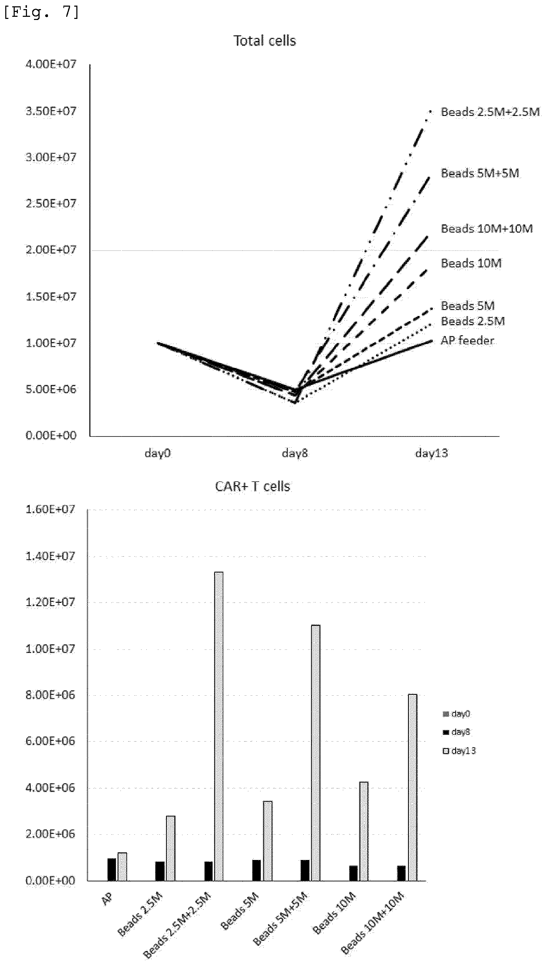

   Fig. 7 presents graphs showing the measurement results of the cell number (upper row) and CAR expression rate (lower row) of CAR-T cells after expansion culture.

### [Description of Embodiments]

The present invention is described below. Unless otherwise specified, the terms used in the present specification have meanings generally used in the pertinent field.

The present invention provides a method for producing a chimeric antigen receptor T (CAR-T) cell. The production method of CAR-T cells of the present invention includes a step of introducing a cancer specific gene (so-called CAR gene) into T cells derived from a T cell source, and expansion culturing the obtained cells.

### T cell and T cell source

In the present invention, the CAR gene is introduced into T cells. The T cells in the present invention include CD4-positive CD8-negative T cells, CD4-negative CD8-positivse T cells, CD4-positive CD8-positive T cells, CD4-negative CD8-negative T cells, αβ-T cells, γδ-T cells, Treg cells, NK-like T cells, and NKT cells, and the like. T cells can be subsets such as naive T cells, effector T cells, or memory T cells. T cells may be cells isolated from humans, or cells obtained by differentiation from cells such as iPS cells, ES cells, hematopoietic stem cells, mesenchymal stem cells, and the like. In addition, the T cells may be either autologous cells or allogeneic cells. In the present invention, "autologous cell" refers to a cell obtained from a subject who receives administration of a cell population (i.e., CAR-T cells) produced by the method of the present invention, or a cell induced from the obtained cell, and "allogeneic cell" means that it is not the aforementioned "autologous cell." Preferably, the T cell is an autologous cell. CAR-T cell can be obtained by gene transfer into a cell population containing the T cell or a progenitor cell thereof such as hematopoietic stem cell. For example, CAR-T cell may be a cell obtained by differentiating cells such as iPS cell, ES cell, hematopoietic stem cell, and mesenchymal stem cell, that have been introduced with the CAR gene, or a cell obtained by differentiating a cell that has been transformed into an iPS cell after introducing the CAR-T gene. CAR-T cells are prepared by introducing a CAR gene into T cells, and in one embodiment, the T cells to which the CAR gene is introduced are T cells that have been bead-separated with a specific factor from a T cell source. The T cell source is not particularly limited as long as it contains T cells that exhibit a specific expression pattern for a specific cell membrane surface antigen (having a specific cell membrane surface marker). Generally, it is a blood cell or a sample derived from blood, or a product derived from apheresis or leukapheresis or derived therefrom. The T cell source in the present invention may be a frozen cell.

In the present invention, the blood cell refers to a cell that constitutes blood, and can be used to refer to a single cell, a cell population containing multiple cells, or a cell population consisting of one type of cell, or also used to refer to a cell population containing multiple types of cells. The blood cell is preferably a blood cell excluding erythrocytes and platelets, and such blood cell includes immune cells such as lymphocytes and monocytes. The blood cell may be a cell isolated from human, a cell obtained by differentiation from cells such as iPS cells, ES cells, hematopoietic stem cells, mesenchymal stem cells, and the like, or may be either an autologous cell or an allogeneic cell, preferably an autologous cell. In a further embodiment, CAR-T cell is prepared by introducing a CAR gene into a peripheral blood mononuclear cell (PBMC). PBMC is preferably an autologous PBMC (i.e., PBMC collected from a subject who receives administration of a cell population produced by the method of the present invention). PBMC can be prepared by conventional methods, for example, Saha S, Nakazawa Y, Huye LE, Doherty JE, Galvan DL, Rooney CM, Wilson MH. J Vis Exp. 2012 Nov 5; (69): e4235. Unless particularly indicated, various cells (e.g., T cells) in the present specification are human cells.

### Chimeric antigen receptor (also referred to as CAR in the present specification)

CAR is a structure that contains, from the N-terminal side to the C-terminal side of a protein, a target-specific extracellular domain, a transmembrane domain, and an intracellular signal domain for effector functions of immune cells, and CAR gene is a gene encoding this receptor. The extracellular domain contains an antigen recognition site that exhibits target-specific binding. The transmembrane domain exists between the extracellular domain and the intracellular signal domain. The intracellular signal domain transmits signals necessary for immune cells to exert their effector functions. That is, when the extracellular domain binds to a target antigen, an intracellular signal domain is used that is capable of transmitting a signal necessary for activating immune cells.

There have been several reports of experiments and clinical studies using CAR, and the CAR of the present invention can be constructed by referring to these reports (e.g., Rossig C, et al. Mol Ther 10:5-18, 2004; Dotti G, et al. Hum Gene Ther 20:1229-1239, 2009; Ngo MC, et al. Hum Mol Genet 20 (R1):R93-99, 2011; Ahmed N, et al. Mol Ther 17:1779-1787, 2009; Pule MA, et al. Nat Med 14:1264-1270, 2008; Louis CU, et al. Blood 118:6050-6056, 2011; Kochenderfer JN, et al. Blood 116:4099-4102, 2010; Kochenderfer JN, et al. Blood 119:2709-2720, 2012; Porter DL, et al. N Engl J Med 365:725-733, 2011; Kalos M, et al. Sci Transl Med 3:95ra73,2011; Brentjens RJ, et al. Blood 118:4817-4828, 2011; Brentjens RJ, et al. Sci Transl Med 5:177 ra38, 2013).

One embodiment of the present invention is characterized in that it includes a step of separating T cells from a T cell source with a specific factor before introducing the CAR gene. In particular, in the present invention, it is preferable to separate T cells from a T cell source with a specific factor, by bead separation before introducing the CAR gene.

The step of separating T cells from a T cell source by using a specific factor is described.

This step is a step of, before introducing the CAR gene, selecting T cells to be targeted for CAR gene introduction, by performing, on a T cell source, a step of separating T cells expressing a specific cell membrane surface antigen from T cells not expressing the antigen, or a step of separating T cells not expressing a specific cell membrane surface antigen from T cells expressing the antigen. That is, the specific factor means that a specific cell membrane surface antigen is expressed (positive, +) or that a specific cell membrane surface antigen is not expressed (negative, -). The specific factor here is not particularly limited as long as it is capable of selecting T cells to be subjected to CAR gene introduction from the T cell source, and may have any expression profile (positive or negative) of cell membrane surface antigen.

Examples of the cell membrane surface antigen are shown in Tables 1-1 to 1-5, but the antigens are not limited thereto.

**[Table 1-1]**

| **factor** | **constitution, another name, etc.** |
|---|---|
| CD3 | CD3δ/CD3ε, CD3γ/CD3ε, CD3γ, CD3δ, CD3ε, T3 |
| CD45RA | - |
| CD27 | TNFRSF7 |
| CD31 | PECAM1 |
| IL-7R | IL-7Rα, CD127 |
| CCR7 | CD197, CMKBR7 |
| CD62L | SELL; LYAM 1, LECAM-1, L-selectin |
| CD95 | TNFRSF6, APT1, Fas, APO-1 |
| ICOS | CD278, AILIM |
| HVEM | - |
| OX40 | CD134 |
| 4-1BB | CD137, TNFRSF9 |
| CD40L | CD154, TNFSF5, TRAP |
| GITR | TNFSF18 |
| IL-15R | IL-15α, IL2/15Rβ, CD122, IL-15Rγ, CD132, CD215 |
| PTGER2 | - |
| ICOSLG | CD275, B7H2 |
| CD38 | T10 |
| CXCR5 | CD185, BLR1 |
| IFNGR2 | - |
| BTLA | CD272 |
| CD28 | Tp44 |
| PD-L1 | CD274, PDCD1LG1 |

**[Table 1-2]**

| | |
|---|---|
| CCL5 | RANTES |
| TNFSF4 | CD252, TXGPL, OX40L |
| XCL1 | Lymphotactin α, SCM-1α, ATAC |
| CX3CR1 | fractalkine receptor, GPR13 |
| CCL3L3 | - |
| CCL4 | - |
| Notch1 | - |
| Notch2 | - |
| CD248 | CD248;TEM1 |
| CD327 | SIGLEC6;CD33L1 |
| CD79a | CD79A;IGA;MB1 |
| CD35 | C3BR;CR1 |
| CD73 | NT5E;NT5;NTE |
| CD318 | CDCP1;TRASK |
| CD305 | LAIR1 |
| CD242 | ICAM4;LW |
| CD158d | KIR2DL4 |
| CD42d | GP5 |
| CD56 | NCAM1;NCAM |
| CD257 | TNFSF13B;BAFF |
| CD49f | ITGA6 |
| CD201 | PROCR;EPCR |
| CD7 | CD7 |
| CD328 | SIGLEC7;AIRM1 |
| CD300c | CD300C;CMRF35A1 |

**[Table 1-3]**

| | |
|---|---|
| CD11d | ITGAD;CD11D |
| CD228 | MFI2;MAP97 |
| CD336 | NCR2;LY95 |
| CD289 | TLR9 |
| CD172b | SIRPB1 |
| CD167a | DDR1;EDDR1;CAK |
| CD353 | SLAMF8;BLAM2 |
| CD355 | CRTAM |
| CD217 | IL17RA;IL17R |
| CD11c | ITGAX;CD11C |
| CD258 | TNFSF14;HVEML |
| CCL19 | - |
| CD80 | B7, BB1 |
| 4-1BBL | TNFSF9, CD137L |
| CD40 | TNFRSF5 |
| CD52 | CAMPATH-1 |
| CD70 | CD27L, TNFSF7 |
| CD49 | CD49a, CD49b, CD49c, CD49d, CD49e, cd49f, CD49g, CD49h |
| CD51 | Integrin α V, VNR α |
| CD103 | αEβ7 |
| CD11b | MAC-1, IntegrinαM, Mo1, CR3 |
| ITGA | ITGA9, ITGA10, ITGA11 |
| CD18 | Integrinβ2, LFA-Iβ |
| CD19 | B4, Bgp95 |
| ITGB | ITGB4, ITGB5, ITGB6, ITGB7, ITGB8 |

**[Table 1-4]**

| | |
|---|---|
| CD57 | Leu-7; HNK-1 |
| CD58 | LFA3 |
| KLRG1 | - |
| LAG3 | - |
| PD-1 | CD279, PDCD1 |
| CD11a | ITGAL, LFA-1 |
| CD26 | DPP4;ADCP2, Tp103, ADA-BP |
| IL-2Rβ | - |
| HLA-DR | - |
| CD86 | CD28-L,CTLA-4L |
| CXCL10 | IP-10, CRG-2 |
| ID2 | - |
| CCL4L2 | - |
| CCL3 | MIP-1α |
| CD249 | ENPEP |
| CD150 | SLAMF1;SLAM |
| CD283 | TLR3 |
| CC3131 | CSF2RB |
| CD66a | CEACAM1 |
| CD253 | TNFSF10 |
| CD120b | TNFRSF1B |
| CD61 | ITGB3 |
| CD87 | PLAUR |
| CD280 | MRC2 |
| CD54 | ICAM1 |

**[Table 1-5]**

| | |
|---|---|
| CD265 | TNFRSF11A |
| CD203a | ENPP1 |
| CD271 | NGFR |
| CD68 | CD68 |
| CD307c | FCRL3 |
| CD357 | TNFRSF18 |
| CD25 | IL2RA |
| CD203c | ENPP3 |
| CD106 | VCAM1 |
| CD244 | CD244; 284 |
| CD71 | TFRC |
| CD220 | INSR |
| CD158e | KIR3DL1;NKAT3 |
| CD123 | IL3RA |
| CD16b | FCGR3B; FCG3 |
| CD364 | PI16;CRISP9 |
| CD230 | PRNP |
| CD41 | ITGAB;GP2B |
| CD361 | EVI2B-EVDB |
| CD161 | KLRB1;CLEC5B |
| CD292 | BMPR1A;ACVRLK3 |

Positive (+) specific factor includes the following. CD3+, CD4+, CD8+, CD45RA+, CD27+, IL-7R+, CCR7+, CD62L+, CD95+, ICOS+, HVEM+, OX40+, CD44+, 4-1BB+, CD40L+, GITR+, IL-15R+, PTGER2+, ICOSLG+, CD38+, CXCR5+, IFNGR2+, BTLA+, CD28+, PD-L1+, CCL5+, TNFSF4+, XCL1+, CX3CR1+, CCL3L3+, CCL4+, Notch1+, Notch2+, CD248+, CD327+, CD79a+, CD35+, CD73+, CD318+, CD305+, CD242+, CD158d+, CD42d+, CD56+, CD257+, CD49f+, CD201+, CD7+, CD328+, CD300c+, CD11d+, CD228+, CD336+, CD289+, CD172b+, CD167a+, CD353+, CD355+, CD217+, CD172g+, CD183+, CD11c+, CD159a+, CD69+, CD366+, CD258+, CD254+, CCL19+, CXCR1+, CXCR2+, CXCR4+, CCR2+, CCR4+, CCR5+, CCR6+, CD6+, CD45RB+, CD45RC+, CD80+, 4-1BBL+, CD40+, CD52+, CD70+, CD49+, CD51+, CD103+, CD11b+, ITGA+, CD18+, CD19+, and ITGB+.

Preferably, CD3+, CD4+, CD8+, CD45RA+, CD27+, IL-7R+, CCR7+, CD62L+, CD95+, CXCR5+, CD28+, CCL5+, CCL19+, CD45RB+, CD45RC+, CD80+, CD44+, and 4-1BBL+ can be mentioned.

Negative (-) specific factor includes the following. CD45RO-, CD57-, CD58-, KLRG1-, TIM3-, LAG3-, PD-1-, CD11a-, CD26-, CD122-, CXCR3-, IL-2Rβ-, HLA-DR-, CD86-, 4-1BBL-, CXCL10-, ID2-, CCL4L2-, CTLA4-, CD160-, CCL3-, CD28-, CD249-, CD150-, CD283-, CD131-, CD66a-, CD253-, CD120b-, CD61-, CD87-, CD280-, CD195-, CD54-, CD265-, CD203a-, CD271-, CD68-, CD307c-, CD357-, CD196-, CD25-, CD154-, CD203c-, CD134-, CD106-, CD244-, CD71-, CD220-, CD158e-, CD123-, CD16b-, CD364-, CD230-, CD41-, CD361-, CD161-, CCR4, and CD292-.

Preferably, CD45RO-, CD57-, LAG3-, CXCR3-, and IL-2Rβ-, can be mentioned.

Preferred specific factor includes CD3+, CD4+, CD8+, CD45RA+, CD27+, IL-7R+, CCR7+, CD62L+, CD95+, CXCR5+, CD28+, CCL5+, CCL19+, CD45RB+, CD45RC+, CD80+, 4-1BBL+, CD44+, CD45RO-, CD57-, LAG3-, CXCR3-, and IL-2Rβ-, more preferably CD45RA+, IL-7R+, CD62L+, CD3+, and CD28+, further preferably CD45RA+ and CD62L+. It is preferable to use CD45RA+ as a specific factor because many CAR-positive cells can be obtained. On the other hand, it is preferable to use CD62L+ as a specific factor because the proportion of positive naive or young memory T cells such as Tscm and the like in T cells or CAR-T cells can be increased.

The step of separating T cells from a T cell source using a specific factor can be performed using a method generally used to separate cells based on the expression status of cell membrane surface antigens. Preferred embodiments of the method of the present invention include density gradient separation method, immunological cell separation technique, magnetic cell separation technique, nylon wool separation method, and adhesion method.

The density gradient separation method (also to be referred to as density gradient centrifugation method) is a technique that allows cells to be separated according to the size, shape, and density thereof. A density gradient can be formed in a container such as a centrifuge tube by layering solutions of various densities that become highly dense at the bottom of a container such as a tube.

The magnetic cell separation technique is also known as a magnetic cell sorting (MACS) method, and involves preparing a cell suspension from tissue containing a mixture of various cells, magnetically labeling specific cells in the suspension, and separating and collecting magnetically labeled cells and non-magnetically labeled cells.

The immunological cell separation technique is a method of separating cells by using antigen-antibody reactions.

The nylon wool separation method is a method that utilizes the property that B cells adhere well to nylon wool.

The adhesion method is a method of separating cells by utilizing differences in the ability of the cells to adhere to a substrate (e.g., culture flask). Specifically, when a T cell source is placed in a culture flask, monocytes etc. that have adhesive ability adhere to the bottom of the culture flask, while lymphocytes such as T cells that do not have adhesive ability float in the culture medium, whereby they are separated into two cell groups using the adhesion method. When cells that adhere to a culture container are removed, a T cell source is left in the culture container for a certain period of time (e.g., 1 second to 7 days), and after lapse of a certain period of time, only the floating cells are collected and used as a T cell source without using the adherent cells. Examples of culture container include, but are not limited to, culture flask, culture chamber, culture bag, culture plate, culture dish, bioreactor, and the like. Furthermore, the material of the culture container is not particularly limited, and polystyrene, TPP, PETG, glass, and the like can be mentioned. The culture container may or may not be coated or surfacetreated with a coating agent (e.g., collagen, fibronectin, polylysine, plasma treatment, electric charge treatment, etc.).

These separation methods and separation techniques may be used alone or two or more kinds of separation techniques may be combined. For example, immune density gradient separation method that combines density gradient separation method and immunological cell separation technique, immune magnetic cell separation technique that combines magnetic cell separation technique and immunological cell separation technique, a technique in which cells are separated by adhering them to a culture container coated with an antibody or the like, by combining adhesion method and immunological cell separation method, and the like can be mentioned. Cell separation using immunological cell separation techniques is performed by labeling cells with antibodies or ligands specific to the surface of the cells to be separated, and performing density gradient fractionation using the label as an indicator. One of the immune magnetic cell separation techniques is magnetic bead separation using magnetic beads (hereinafter to be also simply referred to as bead separation). In the present invention, bead separation is preferred.

### Bead separation

Bead separation uses beads labeled with antibody or ligand specific to the surface of the cells to be separated, contacts a slurry of the beads with a cell suspension, and then removes the cells bound to the beads, by using an affinity capture method that is specific to some property of the beads. This form facilitates both positive selection and negative selection.

The material of the beads used for bead separation can be those generally used in this field, and examples include, but are not limited to, magnetic substances, latex, agarose, glass, cellulose, sepharose, nitrocellulose, polystyrene, and the like. A preferred material is a magnetic substance, and therefore magnetic beads are preferably used for bead separation.

The particle size of the beads varies depending on the material and the affinity capture method used for separation, and is determined as appropriate. In the case of magnetic beads, it is generally 10 nm to 500 µm, preferably 10 nm to 100 µm, more preferably 10 nm to 50 um. When the particle size is too small or too large, the target cells cannot be captured and separation cannot be performed successfully.

Magnetic beads are generally uniform superparamagnetic beads coated affinity tags such as recombinant streptavidin that can bind biotinylated immunoglobulin or other biotinylated molecules, such as, for example, peptides/proteins or lectin. Magnetic beads are generally uniform microparticles or nanoparticles of Fe3O4 (magnetic core).

In one embodiment, the magnetic beads are coated with antibody (preferably monoclonal antibody) against cell surface antigen that may or may not be expressed by the T cells of interest. The antibody-tagged magnetic beads are then introduced into the cell suspension of the T cell source. Magnetic beads bind or do not bind with T cells having a specific factor, and they can be separated by applying a magnetic field. This can be promoted by running the suspension through a high gradient magnetic separation column placed under a strong magnetic field. Magnetically labeled cells remain on the column, while unlabeled cells pass through. When the column is removed from the magnetic field, the magneticallymaintained cells are eluted. Both labeled and unlabeled fractions can be completely recovered.

The magnetic beads used for bead separation are commercially available, or can be produced by applying a desired coating to available magnetic beads themselves using a known method.

### Introduction of CAR gene into T cells (production of CAR-T cells)

In the present invention, CAR-T cells are prepared by introducing a CAR gene into T cells by a CAR expression vector. The T cells into which the CAR gene is introduced are preferably T cells that have been bead-separated using a specific factor from the above-mentioned T cell source and have a characteristic cell surface marker expression pattern. The CAR expression vector means a nucleic acid molecule capable of transporting a nucleic acid molecule encoding a CAR gene into T cells. It is not questioned whether it is DNA or RNA and is not particularly limited as to the form, origin, and the like, and various kinds of vectors can be used. The vector may be a viral vector or a non-viral vector. As the viral vector, retroviral vector, lentivirus vector, adenoviral vector, adeno-associated viral vector, herpes virus vector, sendai virus vector, vaccinia virus vector, poxvirus vector, phage, and the like can be mentioned. Among these, with retroviral vectors, lentivirus vectors, and adeno-associated viral vectors, the target gene incorporated into the vector is integrated into the host chromosome, and stable and long-term expression can be expected. Each viral vector can be produced according to a conventional method or using a commercially available dedicated kit. Non-viral vectors include plasmid vectors, liposome vectors, positively charged liposome vectors (Felgner, P.L., Gadek, T.R., Holm, M. et al., Proc. Natl. Acad. Sci., 84:7413-7417, 1987), YAC vectors, BAC vectors, artificial chromosome vectors, cosmid vectors, and the like.

A CAR expression vector contains an expression unit for expressing a CAR gene, and the expression unit generally contains a promoter, a CAR gene, and a poly A addition signal. The expression unit is derived from various organisms or viruses or is composed of any sequence, and includes similar sequences and modified units thereof. Promoters that can be used in the CAR expression cassette include CAG promoter, CMV-IE (cytomegalovirus early gene-derived promoter), SV40ori, retrovirus LTRSRα, EF1α, β actin promoter, and the like. Examples of the poly A addition signal sequence include poly A addition sequence of SV40, poly A addition sequence of bovinederived growth hormone gene, globulin poly A addition sequence, and the like. In order for the expression of the CAR gene to be controlled by the promoter, the CAR gene is generally linked directly to the 3'-terminal side of the promoter or via other sequence, and a poly A addition signal sequence is configured downstream of the CAR gene. The CAR gene is transcribed into messenger RNA (mRNA) by such expression unit, and CAR is translated from the mRNA and displayed on the cell surface.

The expression unit may contain detection genes (reporter genes, cell- or tissue-specific genes, selection marker genes, and the like) to enable detection of gene expression, an enhancer sequence to improve expression efficiency, a WRPE sequence, and the like.

The detection gene is used for determining the success or failure and efficiency of introduction of the CAR expression vector, detecting the expression of the CAR gene or determining the expression efficiency, and selecting and sorting cells in which the CAR gene is expressed, and the like. As the detection gene, the neo gene that confers resistance to neomycin, kmr gene and nptII gene that confer resistance to kanamycin and the like (Bernd Reiss et al. EMBO J. 3 (1984), 3317-3322), hph gene that confers resistance to hygromycin (Blochlinger & Diggelmann, Mol Cell Bio 4:2929-2931), DHFR gene that confers resistance to methotrexate (Bourouis et al., EMBO J.2(7)), and the like (all marker genes); genes for fluorescent proteins (hereinafter reporter genes) such as luciferase gene (Giacomin, P1. Sci. 116(1996), 59-72; Scikantha, J. Bact. 178(1996), 121), β-glucuronidase (GUS) gene, GFP (Gerdes, FEBS Lett. 389(1996), 44-47) or variants thereof (EGFP, d2EGFP, and the like), and the like; genes such as the epidermal growth factor receptor (EGFR) gene, which lacks an intracellular domain, and the like can be used. The detection gene may be linked to the CAR gene via, for example, a bicistronic control sequence (e.g., internal ribosome entry site (IRES)) or a sequence encoding a self-cleavage peptide. As the self-cleavage peptide, 2A peptide (T2A) derived from Thosea asigna virus can be mentioned. Examples of other self-cleavage peptide include, but are not limited to, 2A peptide (F2A) derived from picornavirus, 2A peptide (F2A) derived from footand-mouth disease virus (FMDV), 2A peptide (E2A) derived from equine rhinitis A virus (ERAV), 2A peptide (P2A) derived from porcine teschovirus (PTV-1), and the like, 2A peptide derived from rotavirus, insect virus, aphthovirus, or trypanosoma virus, and the like. Similar sequences and partially modified sequences can also be mentioned.

The CAR gene expression vector prepared for gene transfer is introduced into T cells by a conventional method. In the case of viral vectors, they are introduced into cells by infection with the virus. In the case of non-viral vectors such as plasmids, conventional methods such as electroporation method, liposomes method, calcium phosphate method, nucleofection method, laser method, cation method, microinjection method, sonoporation, and the like are used for introduction into cells, and electroporation method is preferably used for introduction.

In order to improve the efficiency of integration into the host chromosome, it is preferable to perform gene introduction by the transposon method. The transposon method is one of nonviral gene introduction methods that uses a pair of gene enzyme (transposase) and its specific recognition sequence to cause gene transposition, and can integrate any gene into the host chromosome. As the transposon method, for example, the piggyBac transposon method can be used. The piggyBac transposon method utilizes transposons isolated from insects (Fraser MJ et al., Insect Mol Biol. 1996 May; 5(2):141-51.; Wilson MH et al., Mol THER2007 Jan; 15(1):139-45.), and enables highly efficient integration into mammalian chromosomes. The piggyBac transposon method is actually used for gene introduction (e.g., Nakazawa Y, et al., J Immunother 32:826-836, 2009; Nakazawa Y et al., J Immunother 6:3-10, 2013, and the like).

The transposon method is not limited to one using piggyBac and may also be, for example, a method using transposon, such as Sleeping Beauty (Ivics Z, Hackett PB, Plasterk RH, Izsvak Z (1997) Cell 91: 501-510.), Frog Prince (Miskey C, Izsvak Z, Plasterk RH, Ivics Z (2003) Nucleic Acids Res 31: 6873-6881.), Toll (Koga A, Inagaki H, Bessho Y, Hori H. Mol Gen Genet. 1995 Dec 10; 249(4):400-5.; Koga A, Shimada A, Kuroki T, Hori H, Kusumi J, Kyono-Hamaguchi Y, Hamaguchi S. J Hum Genet. 2007; 52(7):628-35. Epub 2007 Jun 7.), Tol2 (Koga A, Hori H, Sakaizumi M (2002) Mar Biotechnol 4: 6-11.; Johnson Hamlet MR, Yergeau DA, Kuliyev E, Takeda M, Taira M, Kawakami K, Mead PE (2006) Genesis 44: 438-445Choo BG, Kondrichin I, Parinov S, Emelyanov A, Go W, Toh WC, Korzh V (2006) BMC Dev Biol 6: 5.), and the like.

The operation of gene introduction using the transposon method can be performed by a conventional method. For example, for the piggyBac transposon method, a vector carrying a gene encoding piggyBac transposase (transposase plasmid) and a vector having a structure in which the CAR gene expression unit is sandwiched between piggyBac inverted repeat sequences (transposon plasmid) are prepared, and these vectors can be introduced into target cells by various techniques such as electroporation, nucleofection, lipofection, and calcium phosphate method.

One embodiment of the method for producing the CAR-T cell of the present invention characteristically includes a step of activating T cells in the absence of feeder cells. Activation of T cells in the present invention refers to supporting the maintenance and/or proliferation of T cells such as naive T cells, young memory T cells, effector T cells, and the like, and imparting a high antitumor effect when CAR-T cells are prepared. T cells may be activated in the absence of feeder cells before or after (in this case, T cells should be read as CAR-T cells) the CAR gene is introduced into T cells, and preferably activated in both steps.

The treatment before CAR gene introduction is performed in order to sufficiently activate T cells, and the activated T cells can be maintained and/or proliferated in a state susceptible to gene introduction.

The treatment after CAR gene introduction is performed in order to sufficiently activate CAR-T cells, and the activated CAR-T cells can be maintained and/or proliferated in a state where they can easily recognize tumor-associated antigens or tumor-specific antigens to be the targets of CAR, which makes it possible to achieve expansion culture of CAR-T cells to the required scale. Furthermore, the maintenance and/or proliferation of naive or memory T cells among CAR-T cells can be supported and high antitumor effects can be imparted to CAR-T cells. For example, sufficient amounts of CAR-T cells having high antitumor property can be obtained for clinical use.

The method of activation treatment is not particularly limited as long as the desired effect on T cells or CAR-T cells can be obtained. For example, it can be performed by contacting with a stimulating substance. The stimulating substance is a signal molecule that controls cell activity by methods using autocrine, paracrine, or endocrine. It may be a substance that can be secreted from all cells (including cells other than T cells or CAR-T cells) contained in the culture system, or may be added from outside. Culture supernatants from other cells such as mesenchymal stem cells can also be added for the purpose of activating T cells.

### (T cell activation treatment before CAR gene introduction)

Specifically, T cell activation treatment before CAR gene introduction can be performed by contacting with a stimulating substance. The stimulating substance is not particularly limited as long as it specifically stimulates T cells and sufficiently activates the T cells, and examples thereof include proteins, peptide fragments, glycans, and the like. The stimulating substance induces or inhibits the expression of a stimulation factor. The stimulation factors are broadly classified into those that activate T cells by increased expression within cells, or those that activate T cells by promoted expression of stimulation factors within T cells due to the contact with the stimulating substance (positive: stimulation factor (+)), and those that activate T cells by reduced expression within cells, or those that activate T cells by suppresses expression of stimulation factors within T cells due to the contact with the stimulating substance (negative: stimulation factor (-)). The stimulating substance that provides the stimulation factor (+) includes the stimulation factor itself, and the stimulating substance that provides the stimulation factor (-) includes an inhibitor (e.g., antibody) of the stimulation factor.

### Examples of the former stimulation factor (+) include

CD4+, CD8+, CD45RA+, CCR7+, CD62L+, ICOS+, HVEM+, OX40+, 4-1BB+, CD40L+, GITR+, TCF1+, BCL6+, IL-15R+, PTGER2+, ICOSLG+, NFKB1+, SATB1+, CD38+, BACH2+, TCF7+, LEF1+, ID3+, LEF1-AS1+, SATB1-AS1+, NFATC1+, CXCR5+, IFNGR2+, BTLA+, PD-L1+, BCL2L2+, CD101+, CCL5+, TNFSF4+, XCL1+, PRDM15+, BCL2L10+, CX3CR1+, CCL3L3+, CCL4+, carnosine+, vitamin A+, vitamin E+, Ca+, TLAM+, AMPK+, HIF-1+, Dexamethasone+, CD248+, CD327+, CD79a+, CD35+, CD73+, CD318+, CD305+, CD242+, CD158d+, CD42d+, CD56+, CD257+, CD49f+, CD201+, CD7+, CD328+, CD300c+, CD11d+, CD228+, CD336+, CD289+, CD172b+, CD167a+, CD353+, CD355+, CD217+, CD172g+, CD183+, CD11c+, CD159a+, CD69+, CD366+, CD258+, CD254+, CCL19+, CXCR1+, CXCR2+, CXCR4+, CCR2+, CCR4+, CCR5+, CCR6+, CD6+, CD45RB+, CD45RC+, CD40+, CD52+, CD70+, CD49+, CD51+, CD103+, CD11b+, ITGA+, CD18+, CD19+, and ITGB+.

Preferred examples include CD4+, CD8+, CD45RA+, CCR7+, CD62L+, CXCR5+, CCL5+, CD327+, CD73+, CCL19+, CD45RB+, and CD45RC+, more preferably CCR7+, CD62L+, CXCR5+, CCL5+, CD327+ and CD73+, further preferably CD62L+, CD327+ and CD73+, most preferably CD62L+.

Each stimulation factor is summarized in Tables 2-1 to 2-4.

**[Table 2-1]**

| **factor** | **constitution, another name, etc.** |
|---|---|
| CD4 | leu-3, T4 |
| CD8 | CD8α, CD8β |
| CD45RA | - |
| CD45RO | - |
| CCR7 | CD197, CMKBR7 |
| CD62L | SELL;LYAM1, LECAM-1, **L-selectin** |
| ICOS | CD278, AILIM |
| HVEM | - |
| OX40 | CD134, TNFRSF4 |
| 4-1BB | CD137, TNFRSF9 |
| CD40L | CD154, TNFSF5, TRAP |
| GITR | TNFSF18 |
| TCF1 | - |
| BCL6 | - |
| IL-15R | IL-15α, IL2/15Rp, CD122, IL-15Rγ, CD132, CD215 |
| PTGER2 | - |
| ICOSLG | CD275, B7H2 |
| NFKB1 | - |
| SATB1 | - |
| CD38 | T10 |
| BACH2 | - |
| TCF7 | - |
| LEF1 | - |
| ID3 | - |
| LEF1-AS1 | - |
| SATB1-AS1 | - |
| NFATC1 | - |
| CXCR5 | CD185, BLR1 |

**[Table 2-2]**

| | |
|---|---|
| IFNGR2 | - |
| BTLA | CD272 |
| PD-L1 | CD274, PDCD1LG1 |
| BCL2L2 | - |
| CD101 | IGSF2 |
| CCL5 | RANTES |
| TNFSF4 | CD252, TXGP1, OX40L |
| XCL1 | Lymphotactin α, SCM-1α, ATAC |
| PRDM15 | - |
| BCL2L10 | - |
| BCL2L11 | - |
| BCL2L14 | - |
| BCL2A1 | - |
| BCL2L15 | - |
| CX3CR1 | fractalkine receptor, GPR13 |
| CCL3L3 | - |
| CTLA4 | CD152 |
| CCL4 | - |
| **carnosine** | - |
| Rapamycin | - |
| vitaminA | - |
| vitaminE | - |
| Ca | - |
| Notch1 | - |
| Notch2 | - |
| TLAM | - |
| AMPK | - |
| HIF-1 | - |
| Dexamethasone | - |
| CD248 | CD248;TEM1 |

**[Table 2-3]**

| | |
|---|---|
| CD327 | SIGLEC6;CD33L1 |
| CD79a | CD79A;IGA;MB1 |
| CD35 | C3BR;CR1 |
| CD73 | NT5E;NT5;NTE |
| CD318 | CDCP1;TRASK |
| CD305 | LAIR1 |
| CD242 | ICAM4;LW |
| CD158d | KIR2DL4 |
| CD42d | GP5 |
| CD56 | NCAM1;NCAM |
| CD257 | TNFSF13B;BAFF |
| CD49f | ITGA6 |
| CD201 | PPROCR;EPCR |
| CD7 | CD7 |
| CD328 | SIGLEC7;AIRM1 |
| CD300c | CD300C;CMRF35Al |
| CD11d | ITGAD;CD11D |
| CD228 | MFI2;MAP97 |
| CD336 | NCR2;LY95 |
| CD289 | TLR9 |
| CD172b | SIRPB1 |
| CD167a | DDR1;EDDR1;CAK |
| CD353 | SLAMF8;BLAM2 |
| CD355 | CRTAM |
| CD217 | IL17RA;IL17R |
| CD172g | SIRPG;SIRPB2 |
| CD11c | ITGAX;CD11C |
| CD159a | KLRC1;NKG2A |
| CD69 | Leu-23, MLR-3, AIM,EA1 |
| CD366 | HAVCR2;TIM3; |

**[Table 2-4]**

| | |
|---|---|
| CD258 | TNFSF14;HVEML |
| CD254 | TNFSF11;OPGL |
| CCL19 | - |
| CXCR1 | - |
| CXCR2 | - |
| CXCR4 | - |
| CCR2 | - |
| CCR4 | - |
| CCR5 | - |
| CCR6 | - |
| CD6 | CD6 |
| CD45RB | - |
| CD45RC | - |
| CD40 | TNFRSF5 |
| CD52 | CAMPATH-1 |
| CD70 | CD27L, TNFSF7 |
| CD49 | CD49a, CD49b, CD49c, CD49d, CD49e, cd49f, CD49g, CD49h |
| CD51 | Integrin α V, VNR α |
| CD103 | αEβ7 |
| CD11b | MAC-1, IntegrinaM, Mo1, CR3 |
| ITGA | ITGA9, ITGA10, ITGA11 |
| CD18 | Integrinβ2, LFA-1β |
| CD19 | B4, Bgp95 |
| ITGB | ITGB4, ITGB5, ITGB6, ITGB7, ITGB8 |
| TIM3 | CD366, HAVCR2 |

### Examples of the latter stimulation factor (-) include

CD3-, CD45RO-, CD57-, CD58-, CD44-, KLRG1-, Perforin-, GranzymeA-, GranzymeB-, TIM3-, LAG30-, PD-1-, CD11a-, CD26-, CD122-, CXCR3-, IL-2Rβ-, HLA-DR-, PRDM1-, CD86-, 4-1BBL-, CXCL10-, ID2-, RUNX1-, TIGIT-, CCL4L2-, CTLA4-, CSF1-, BATF-, BCL2-, IFNG-, BATF3-, CCL3-, BCL2L11-, BCL2L14-, BCL2A1-, BCL2L15-, MEK1-, MEK2-, GSK3β-, AKT-, mTOR-, c-Myc-, GLS1-, PPAR-, statin-, PFK1-, IRBIT-, Wnt-, p16-, p21-, CD28-, CD249-, CD150-, CD283-, CD131-, CD66a-, CD253-, CD120b-, CD61-, CD87-, CD280-, CD195-, CD54-, CD265-, CD203a-, CD271-, CD68-, CD307c-, CD357-, CD196-, CD25-, CD154-, CD203c-, CD134-, CD106-, CD244-, CD71-, CD220-, CD158e-, CD123-, CD16b-, CD364-, CD230-, CD41-, CD361-, CD161-, CCR4-CD292-, and IL-21-.

Preferred examples include CD3-, CD45RO-, CD57-, LAG3-, CXCR3-, IL-2Rβ-, BCL2L14-, MEK1-, MEK2-, mTOR-, PPAR-, statin-, CD28-, and IL-21-, more preferably MEK1-, MEK2-, mTOR-, PPAR-, and statin-. Specifically, SB431542-, PD0325901- are preferred as MEK1-, MEK2-, Rapamycin- is preferred as mTOR-, GW9662-, Rosiglitazone-, GW6471- are preferred as PPAR-, and simvastatin- is preferred as statin-.

Each stimulation factor is summarized in Table 3-1 to Table 3-4.

**[Table 3-1]**

| **factor** | **constitution, another name, etc.** |
|---|---|
| CD3 | CD3δ/CD3ε, CD3γ/CD3ε, CD3γ, CD3δ, CD3ε, T3 |
| CD57 | Leu-7; HNK-1 |
| CD58 | LFA3 |
| CD44 | LHR. PGP-1, H-CAM |
| KLRG1 | - |
| Perforin | - |
| GranzymeA | - |
| GranzymeB | - |
| LAG 3 | - |
| PD-1 | CD279, PDCD1 |
| ICOSLG | CD275, B7H2 |
| CD11a | ITGAL, LFA-1 |
| CD26 | DPP4;ADCP2, Tp103, ADA-BP |
| CD69 | Leu-23, MLR-3, AIM,EA1 |
| CD122 | IL-2Rβ |
| CXCR3 | CD183, GPR9 |
| HLA-DR | - |
| PROM1 | - |
| CD86 | CD28-L,CTLA-4L |
| CXCL10 | IP-10, CRG-2 |
| ID2 | - |

**[Table 3-2]**

| | |
|---|---|
| RUNX1 | - |
| TIGIT | - |
| CCL4L2 | - |
| CCL3L3 | - |
| CTLA4 | CD152 |
| ICOS | CD278, AILIM |
| CSF1 | - |
| BATF | - |
| BCL2 | - |
| IFNG | - |
| BATF3 | - |
| CCL3 | MIP-1α |
| CCL4 | - |
| BCL2L11 | - |
| BCL2L14 | - |
| BCL2A1 | - |
| BCL2L15 | - |
| CX3CR1 | GPR13 |
| **MEK1 inhibition** | - |
| **MEK2 inhibition** | - |
| **GSK3β inhibition** | - |
| **AKT inhibition** | - |
| **mTOR inhibition** | - |
| **c-Myc inhibition** | - |
| **GLS1 inhibition** | - |

**[Table 3-3]**

| | |
|---|---|
| **PPAR inhibition** | - |
| **statin inhibition** | - |
| PFK1 | - |
| IRBIT | - |
| **Wnt inhibition** | - |
| **p16 inhibition** | - |
| **p21 inhibition** | - |
| CD28 | Tp44 |
| CD249 | ENPEP |
| CD150 | SLAMF1 |
| CD283 | TLR3 |
| CD131 | CSF2RB |
| CD66a | CEACAM1 |
| CD253 | TNFSF10 |
| CD120b | TNFRSF1B |
| CD61 | ITGB3 |
| CD87 | PLAUR |
| CD280 | MRC2 |
| CD195 | CCR5 |
| CD54 | ICAM1 |
| CD265 | TNFRSF11A |
| CD203a | ENPP1 |
| CD271 | NGFR |
| CD68 | CD68 |
| CD307c | FCRL3 |

**[Table 3-4]**

| | |
|---|---|
| CD357 | TNFRSF18 |
| CD196 | CCR6 |
| CD25 | IL2RA |
| CD154 | CD40LG |
| CD203c | ENPP3 |
| CD254 | TNFSF11 |
| CD106 | VCAM1 |
| CD244 | CD244;2B4 |
| CD71 | TFRC |
| CD159a | KLRC1;NKG2A |
| CD220 | INSR |
| CD158e | KIR3DL1;NKAT3 |
| CD123 | IL3RA |
| CD16b | FCGR3B;FCG3 |
| CD364 | PI16;CRISP9 |
| CD6 | CD6 |
| CD230 | PRNP |
| CD41 | ITGAB;GP2B |
| CD361 | EVI2B;EVDB |
| CD172g | SIRPG;SIRPB2 |
| CD161 | KLRB1;CLEC5B |
| CXCR1 | - |
| CXCR2 | - |
| CXCR4 | - |
| CCR2 | - |
| CCR4 | - |
| CD292 | BMPR1A;ACVRLK3 |
| IL-21 | |

By this treatment, the survival (maintenance) and proliferation of T cells are promoted. By this treatment, moreover, the survival (maintenance) and proliferation of T cells in a state susceptible to gene introduction are promoted. Furthermore, the maintenance and/or proliferation of T cells such as naive T cells, memory T cells, effector T cells, and the like can be supported, and a high antitumor effect can be imparted when CAR-T cells are prepared.

The activation treatment of T cells before gene introduction is optional and, in some cases, a CAR gene can also be directly introduced into a T cell source.

As the treatment after CAR gene introduction, CAR-T cells are expansion cultured to a necessary scale. The expansion culture in the production method of CAR-T cells of the present invention includes an activation treatment to maintain and/or proliferate CAR-T cells in a state where they can easily recognize tumor-associated antigens or tumor-specific antigens to be the targets of CAR and an antigen recognition treatment that imparts CAR-T cells with an antitumor property. The production method of CAR-T cells of the present invention is characterized in that the aforementioned expansion culture is performed in the absence of feeder cells.

### (CAR-T cell activation treatment after CAR gene introduction)

CAR-T cell activation treatment after CAR gene introduction can be performed by contacting with a stimulating substance. The stimulating substance is not particularly limited as long as it specifically stimulates CAR-T cells and sufficiently activates the CAR-T cells, and examples thereof include proteins, peptide fragments, glycans, and the like. The stimulating substance induces or inhibits the expression of a stimulation factor. That is, the stimulation factors are broadly classified into those that activate CAR-T cells by increased expression within cells, or those that activate CAR-T cells by promoted expression of stimulation factors within CAR-T cells due to the contact with the stimulating substance (positive: stimulation factor (+)), and those that activate CAR-T cells by reduced expression within cells, or those that activate CAR-T cells by suppresses expression of stimulation factors within CAR-T cells due to the contact with the stimulating substance (negative: stimulation factor (-)). The stimulating substance that provides the stimulation factor (+) includes the stimulation factor itself, and the stimulating substance that provides the stimulation factor (-) includes an inhibitor (e.g., antibody) of the stimulation factor.

The former stimulation factors (+) are the same as the factors in the activation treatment of T cells and summarized in Tables 2-1 to 2-4. Preferred examples include CD4+, CD8+, CD45RA+, CCR7+, CD62L+, CXCR5+, CCL5+, CD327+, CD73+, CCL19+, CD45RB+, and CD45RC+, more preferably CCR7+, CD62L+, CXCR5+, CCL5+, CD327+ and CD73+, further preferably CD62L+, CD327+ and CD73+, most preferably CD62L+.

The latter stimulation factors (-) are the same as the factors in the activation treatment of T cells and summarized in Tables 3-1 to 3-4. Preferred examples include CD3-, CD45RO-, CD57-, LAG3-, CXCR3-, IL-2Rβ-, BCL2L14-, MEK1-, MEK2-, mTOR-, PPAR-, and statin-, more preferably MEK1-, MEK2-, mTOR-, PPAR-, and statin-. Specifically, SB431542-, PD0325901- are preferred as MEK1-, MEK2-, Rapamycin- is preferred as mTOR-, GW9662-, Rosiglitazone-, GW6471- are preferred as PPAR-, and simvastatin- is preferred as statin-.

By this treatment, the survival (maintenance) and proliferation of CAR-T cells are promoted. By this treatment, moreover, the maintenance and/or proliferation of CAR-T cells in a state where they can easily recognize tumor-associated antigens or tumor-specific antigens to be the targets of CAR is promoted, and the maintenance and/or proliferation of naive or young memory T cells in CAR-T cells can afford a sufficient amount of CAR-T cells having high antitumor property.

The activation treatment of T cells or CAR-T cells can also be explained as follows.

The activation treatment of T cells or CAR-T cells (hereinafter also to be referred to as "T cells, etc.") can be performed by contacting T cells, etc. with a stimulating substance. The stimulating substances are broadly classified into (1) substances that induce and/or promote signal transduction that promote activation of T cells, etc., and (2) substances that suppress signal transduction that inhibit activation of T cells, etc. In the present specification, stimulating substances of (1) and (2) are sometimes referred to as stimulating substance (+) and stimulating substance (-), respectively. The induction and/or promotion or inhibition of signal transduction by the stimulating substance (+) or the stimulating substance (-) may be achieved at any stage from upstream to downstream in signal transduction as long as the desired effect is obtained.

In one embodiment, the stimulating substance (+) may be, but is not limited to, the following:
CD4+ —> anti-CD4 antibody
CD8+ —> anti-CD8 antibody
CD45RA+ → anti-CD45RA antibody, anti-CD62L antibody, anti-CXCR5 antibody, anti-CCL5 antibody, anti-CCR7 antibody, anti-CD73 antibody, anti-CD327 antibody, anti-CD4 antibody, anti-TIM3 antibody, anti-CD45RO antibody, anti-CCL19 antibody, anti-4-1BB antibody, anti-CD8 antibody, anti-CD45RB antibody, anti-CD44RO antibody, anti-CD79a antibody, anti-CD80 antibody, anti-CD248 antibody, GW9662, Rosiglitazone, GW6471, simvastatin, SB431542, Rapamycin, PD0325901, catechin
CCR7+ -> anti-CCR7 antibody, anti-CD62L antibody, anti-CXCR5 antibody, anti-CCL5 antibody, anti-CD73 antibody, anti-CD327 antibody, anti-CD4 antibody, anti-TIM3 antibody, anti-CD45RO antibody, anti-CCL19 antibody, anti-4-1BB antibody, anti-CD8 antibody, anti-CD45RB antibody, anti-CD44RO antibody, anti-CD79a antibody, anti-CD80 antibody, anti-CD248 antibody, GW9662, Rosiglitazone, GW6471, simvastatin, SB431542, Rapamycin, PD0325901, catechin
CD62L+ —> anti-CD62L antibody, GlyCAM-1 protein, CD34 protein, PSGL-1 protein
ICOS+ —> anti-ICOS antibody
HVEM+ —> anti-HVEM antibody, CD258 protein
OX40+ —> anti-OX40 antibody
4-1BB+ —> anti-4-1BB antibody
CD40L+ —> anti-CD40L antibody, CD40 protein
GITR+ —> anti-GITR antibody
TCF1+ —> one that increases expression of transcription factor TCF1, PPAR inhibitor (GW6471, Rosiglitazone, GW9662, pemafibrate, ezetimibe, fenofibrate, TPST-1120, fenofibrate, simvastatin, gemfibrozil, fenofibrate, aleglitazar, NS-220, fenofibric acid, rosuvastatin, tesaglitazar, fenofibrate, pravastatin, pirinixic acid, choline fenofibrate, docosahexaenoic acid, clofibrate, atorvastatin, choline fenofibrate, treprostinil, bocidelpar, REN001, icosapent, GW501516, T3D-959, bezafibrate, peroxisome proliferatoractivated receptor gamma agonist, glimepiride, rosiglitazone, clopidogrel, telmisartan, pioglitazone, metformin, rosiglitazone, sulfonylurea, balsalazide, metformin, pioglitazone, sulfonylurea, PPAR gamma inhibitor, inolitazone, telmisartan, INS, metformin, pioglitazone, exenatide, INS, pioglitazone, rosiglitazone, alogliptin, metformin, pioglitazone, exenatide, INS, metformin, pioglitazone, amlodipine, telmisartan, mesalamine, INS, pioglitazone, pioglitazone, sulfonylurea, glimepiride, pioglitazone, metformin, pioglitazone, hydrochlorothiazide, telmisartan, icosapent, aspirin, dipyridamole, telmisartan, troglitazone, alogliptin, pioglitazone, farglitazar, sulfasalazine, nicotinic acid, pioglitazone, rosiglitazone-metformin combination, aleglitazar, GED-0507-34-levo, catechin, which stops the upstream of TCF1, is also effective), Ro41-5253, guggulsterone BCL6+ —> one that increases expression of transcription factor BCL6, PPAR inhibitor (GW6471, Rosiglitazone, GW9662, pemafibrate, ezetimibe, fenofibrate, TPST-1120, fenofibrate, simvastatin, gemfibrozil, fenofibrate, aleglitazar, NS-220, fenofibric acid, rosuvastatin, tesaglitazar, fenofibrate, pravastatin, pirinixic acid, choline fenofibrate, docosahexaenoic acid, clofibrate, atorvastatin, choline fenofibrate, treprostinil, bocidelpar, REN001, icosapent, GW501516, T3D-959, bezafibrate, peroxisome proliferatoractivated receptor gamma agonist, glimepiride, rosiglitazone, clopidogrel, telmisartan, pioglitazone, metformin, rosiglitazone, sulfonylurea, balsalazide, metformin, pioglitazone, sulfonylurea, PPAR gamma inhibitor, inolitazone, telmisartan, INS, metformin, pioglitazone, exenatide, INS, pioglitazone, rosiglitazone, alogliptin, metformin, pioglitazone, exenatide, INS, metformin, pioglitazone, amlodipine, telmisartan, mesalamine, INS, pioglitazone, pioglitazone, sulfonylurea, glimepiride, pioglitazone, metformin, pioglitazone, hydrochlorothiazide, telmisartan, icosapent, aspirin, dipyridamole, telmisartan, troglitazone, alogliptin, pioglitazone, farglitazar, sulfasalazine, nicotinic acid, pioglitazone, rosiglitazone-metformin combination, aleglitazar, GED-0507-34-levo, catechin, which stops the upstream of BCL6, is also effective), Ro41-5253, guggulsterone IL-15R+→ anti-IL-15R antibody, IL-15
PTGER2+→ anti-PTGER2 antibody
ICOSLG+→ anti-ICOSLG antibody, ICOS protein
NFKB1+ —> one that increases expression of transcription factor NFKB1, LTα1α2, BAFF, growth factor FGF, IGF, EGF, PDGF, HGF, BDNF, and the like, TNF-α, IL-1, Ca, DTA-1, catechin
SATB1+ → one that increases expression of transcription factor SATB1, TGFβR inhibitor SB431542, YL-13027, galunisertib, SB-505124, SH3051, vactosertib, IN1233, PF-06952229, SJN2511, SB-525334, SM1-71, GFH018, IL-23
CD38+ —> anti-CD38 antibody
BACH2+ —> one that increases expression of transcription factor BACH2, IL-1β, IFNγ
TCF7+ —> one that increases expression of transcription factor TCF7, PPAR inhibitor (GW6471, Rosiglitazone, GW9662, pemafibrate, ezetimibe, fenofibrate, TPST-1120, fenofibrate, simvastatin, gemfibrozil, fenofibrate, aleglitazar, NS-220, fenofibric acid, rosuvastatin, tesaglitazar, fenofibrate, pravastatin, pirinixic acid, choline fenofibrate, docosahexaenoic acid, clofibrate, atorvastatin, choline fenofibrate, treprostinil, bocidelpar, REN001, icosapent, GW501516, T3D-959, bezafibrate, peroxisome proliferatoractivated receptor gamma agonist, glimepiride, rosiglitazone, clopidogrel, telmisartan, pioglitazone, metformin, rosiglitazone, sulfonylurea, balsalazide, metformin, pioglitazone, sulfonylurea, PPAR gamma inhibitor, inolitazone, telmisartan, INS, metformin, pioglitazone, exenatide, INS, pioglitazone, rosiglitazone, alogliptin, metformin, pioglitazone, exenatide, INS, metformin, pioglitazone, amlodipine, telmisartan, mesalamine, INS, pioglitazone, pioglitazone, sulfonylurea, glimepiride, pioglitazone, metformin, pioglitazone, hydrochlorothiazide, telmisartan, icosapent, aspirin, dipyridamole, telmisartan, troglitazone, alogliptin, pioglitazone, farglitazar, sulfasalazine, nicotinic acid, pioglitazone, rosiglitazone-metformin combination, aleglitazar, GED-0507-34-levo, catechin, which stops the upstream of TCF7, is also effective), Ro41-5253, guggulsterone LEF1+ —> one that increases expression of transcription factor LEF1, PPAR inhibitor (GW6471, Rosiglitazone, GW9662, pemafibrate, ezetimibe, fenofibrate, TPST-1120, fenofibrate, simvastatin, gemfibrozil, fenofibrate, aleglitazar, NS-220, fenofibric acid, rosuvastatin, tesaglitazar, fenofibrate, pravastatin, pirinixic acid, choline fenofibrate, docosahexaenoic acid, clofibrate, atorvastatin, choline fenofibrate, treprostinil, bocidelpar, REN001, icosapent, GW501516, T3D-959, bezafibrate, peroxisome proliferatoractivated receptor gamma agonist, glimepiride, rosiglitazone, clopidogrel, telmisartan, pioglitazone, metformin, rosiglitazone, sulfonylurea, balsalazide, metformin, pioglitazone, sulfonylurea, PPAR gamma inhibitor, inolitazone, telmisartan, INS, metformin, pioglitazone, exenatide, INS, pioglitazone, rosiglitazone, alogliptin, metformin, pioglitazone, exenatide, INS, metformin, pioglitazone, amlodipine, telmisartan, mesalamine, INS, pioglitazone, pioglitazone, sulfonylurea, glimepiride, pioglitazone, metformin, pioglitazone, hydrochlorothiazide, telmisartan, icosapent, aspirin, dipyridamole, telmisartan, troglitazone, alogliptin, pioglitazone, farglitazar, sulfasalazine, nicotinic acid, pioglitazone, rosiglitazone-metformin combination, aleglitazar, GED-0507-34-levo, catechin, which stops the upstream of LEF, is also effective), Ro41-5253, guggulsterone ID3+ → TGFβ
LEF1-AS1+→ LEF1-AS1
SATB1-AS1+→ SATB1-AS1
NFATC1+→ one that increases expression of transcription factor NFATC1, RANKL, NFKB
CXCR5+ -> anti-CXCR5 antibody
IFNGR2+-> anti-IFNGR2 antibody
BTLA+ -> anti-BTLA antibody, HVEM protein
PD-L1+ → anti-PD-L1 antibody
BCL2L2+→ one that increases expression of anti-apoptosis protein BCL2L2, dolastatin 15
CD101+ → anti-CD101 antibody
CCL5+ → anti-CCL5 antibody
TNFSF4+→ anti-TNFSF4 antibody
XCL1+ → anti-XCL1 antibody
PRDM15+→ one that increases expression of transcription factor PRDM15
BCL2L10+→ one that increases expression of anti-apoptosis protein BCL2L10, dolastatin 15
CX3CR1+-> anti-CX3CR1 antibody
CCL3L3+-> anti-CCL3L3 antibody
CCL4+ -> anti-CCL4 antibody
carnosine+ → carnosine
Rapamycin+→ Rapamycin
vitamin A+→ vitamin A
vitamin E+→ vitamin E
Ca+ -> Ca, calcium ion, anti-CD20 antibody
TLAM+ → TLAM
AMPK+ → AMPK
HIF-1+ → one that increases expression of transcription factor HIF-1
CD248+ -> anti-CD248 antibody
CD327+ -> anti-CD327 antibody
CD79a+ -> anti-CD79a antibody
CD35+ -> anti-CD35 antibody
CD73+ -> anti-CD73 antibody
CD318+ -> anti-CD318 antibody
CD305+ -> anti-CD305 antibody
CD242+ -> anti-CD242 antibody
CD158+ —> anti-CD158 antibody
CD42d+ -> anti-CD42d antibody
CD56+ -> anti-CD56 antibody
CD257+ → anti-CD257 antibody
CD49f+ → anti-CD49f antibody, anti-CD29 antibody
CD201+ → anti-CD201 antibody, protein C
CD7+ -> anti-CD7 antibody
CD328+ → anti-CD328 antibody
CD300c+→ anti-CD300c antibody
CD11d+ → anti-CD11d antibody
CD228+ → anti-CD228 antibody
CD336+ → anti-CD336 antibody
CD289+ → anti-CD289 antibody
CD172b+→ anti-CD172b antibody
CD167a+-> anti-CD167a antibody
CD353+ —> anti-CD353 antibody
CD355+ -> anti-CD355 antibody
CD217+ —> anti-CD217 antibody, IL-17
CD172g+→ anti-CD172g antibody
CD11c+ —> anti-CD11c antibody
CD159a+→ anti-CD159a antibody, anti-CD94 antibody
CD69+ —> anti-CD69 antibody
CD258+ → anti-CD258 antibody, HVEM protein
CD254+ -> anti-CD254 antibody
CCL19+ —> anti-CCL19 antibody
CXCR1+ —> anti-CXCR1 antibody
CXCR2+ -> anti-CXCR2 antibody
CXCR4+ -> anti-CXCR4 antibody
CCR2+ -> anti-CCR2 antibody
CCR4+ -> anti-CCR4 antibody
CCR5+ -> anti-CCR5 antibody
CCR6+ -> anti-CCR6 antibody
CD6+ -> anti-CD6 antibody
CD45RB+-> anti-CD45RB antibody
CD45RC+-> anti-CD45RC antibody
CD40+ -> anti-CD40 antibody
CD52+ —> anti-CD52 antibody
CD70+ -> anti-CD70 antibody, CD27 protein
CD49+ -> anti-CD49 antibody, anti-CD29 antibody, laminin, collagen, fibronectin
CD51+ -> anti-CD51 antibody, vitronectin
CD103+ → anti-CD103 antibody
CD11b+ → anti-CD11b antibody
ITGA+ —> anti-ITGA antibody
CD18+ —> anti-CD18 antibody
CD19+ —> anti-CD19 antibody
ITGB+ —> anti-ITGB antibody
TIM3+ —> anti-TIM3 antibody, Ceacam-1, PtdSer, HMGB1
CD45RO+→ anti-CD45RO antibody
CD44+ -> anti-CD44 antibody, hyaluronic acid
Dexamethasone+→ Dexamethasone

In another embodiment, the stimulating substance (-) may be, but is not limited to, the following:
CD3- -> one that prevents signal transduction due to stimulation or inhibits signal transduction, for example, mTOR inhibitor ((Rapamycin, Everolimus, Temsirolimus, everolimus, fulvestrant, apitolisib, everolimus, pasireotide, corticosteroid, sirolimus, PF-4691502, PP-121, GNE-493AZD8055, dactolisib, PKI-179, onatasertib, samotolisib, SF2523, everolimus, letrozole, HEC68498, everolimus, prednisone, everolimus, gefitinib, PI-540, methotrexate, sirolimus, tacrolimus, prednisone, tacrolimus, PI-620, methylprednisolone, tacrolimus, cyclosporine A, sirolimus, tacrolimus, PWT33597, mTOR inhibitor, everolimus, panobinostat, everolimus, sorafenib, everolimus, vandetanib, ETP-45658, WXFL10030390, bimiralisib, pimecrolimus, panulisib, DS-7423, everolimus, exemestane, PKI-402, everolimus, ribociclib, gedatolisib, lenalidomide, temsirolimus, OSI-027, VS-5584, everolimus, tamoxifen, ridaforolimus, everolimus, lenvatinib, vistusertib, everolimus, paclitaxel, mTOR inhibitor, tyrosine kinase inhibitor, ME-344, tacrolimus, imatinib, sirolimus), SB431542, YL-13027, galunisertib, SB-505124, SH3051, vactosertib, IN1233, PF-06952229, SJN2511, SB-525334, SM1-71, GFH018, which stop TGFβR upstream of mTOR), FK506, NFAT inhibitor, Lerdelimumab, Metelimumab, GC-1008, LY550410, SB-505124, SD-208), MEK inhibitor ((PD0325901, Trametinib Dimethyl Sulfoxide, Binimetinib, Selumetinib Sulfate, binimetinib, vemurafenib, ARRY-424704, cobimetinib, encorafenib, dabrafenib, trametinib, dabrafenib, pembrolizumab, trametinib, TAK 733, binimetinib, encorafenib, panitumumab, E 6201, refametinib, PD184352, binimetinib, trametinib, dabrafenib, ipilimumab, trametinib, RO4927350, cobimetinib, atezolizumab, cobimetinib, vemurafenib, U0126, pimasertib, trametinib, vemurafenib, AS703988, dabrafenib, trametinib, vemurafenib, docetaxel, selumetinib, SM1-71, binimetinib, cetuximab, encorafenib, MKK1 inhibitor, mirdametinib, cetuximab, dabrafenib, trametinib, PD318088, cobimetinib, vemurafenib, cobimetinib, dabrafenib, binimetinib, dabrafenib, selumetinib, binimetinib, encorafenib, FCN-159, trametinib, vorinostat), PD98059, U0126, FR180204),
CD57- -> one that prevents signal transduction due to stimulation or inhibits signal transduction
CD58- -> one that prevents signal transduction due to stimulation or inhibits signal transduction
KLRG1- —> one that prevents signal transduction due to stimulation or inhibits signal transduction, E-cadherin
Perforin- → one that prevents signal transduction due to stimulation or inhibits signal transduction
GranzymeA-→ one that prevents signal transduction due to stimulation or inhibits signal transduction
GranzymeB-→ one that prevents signal transduction due to stimulation or inhibits signal transduction, GranzymeB inhibitor Ac-IEPD-CHO, caspase 8 inhibitor Ac-IETD-CHO, Z-IETD-FMK, Ac-AAVALLPAVLLALLAP-IETD-CHO
LAG3- —> one that prevents signal transduction due to stimulation or inhibits signal transduction
PD-1- —> one that prevents signal transduction due to stimulation or inhibits signal transduction
CD11a- —> one that prevents signal transduction due to stimulation or inhibits signal transduction, ICAM2, ICAM3 CD26- —> one that prevents signal transduction due to stimulation or inhibits signal transduction
CD122- —> one that prevents signal transduction due to stimulation or inhibits signal transduction
CXCR3- —> one that prevents signal transduction due to stimulation or inhibits signal transduction
IL-2Rβ- → one that prevents signal transduction due to stimulation or inhibits signal transduction
HLA-DR- → one that prevents signal transduction due to stimulation or inhibits signal transduction
PRDM1- —> one that lowers expression of transcription factor PRDM1, PAX5, FOX, H3K4me1, H3K9me2
CD86- -> one that prevents signal transduction due to stimulation or inhibits signal transduction
CXCL10- —> one that prevents signal transduction due to stimulation or inhibits signal transduction
ID2- —> AKT inhibitor (catechin, which stops the upstream of AKT, is effective)SH-5, Perifosine, Triciribine, TAT-Akt-in, Akt-l-1, 2, API-59CJ-OMe, Akt- inhibitor-X
RUNX1- —> one that lowers expression of transcription factor RUNX1, VEGF
TIGIT- —> one that prevents signal transduction due to stimulation or inhibits signal transduction
CCL4L2- —> one that prevents signal transduction due to stimulation or inhibits signal transduction
CTLA4- —> one that prevents signal transduction due to stimulation or inhibits signal transduction
CSF1- —> one that prevents signal transduction due to stimulation or inhibits signal transduction
BATF- —> one that lowers expression of transcription factor BATF, TGFβR inhibitor SB431542, YL-13027, galunisertib, SB-505124, SH3051, vactosertib, IN1233, PF-06952229, SJN2511, SB-525334, SM1-71, GFH018, AKT inhibitor (catechin, which stops the upstream of AKT, is effective)SH-5, Perifosine, Triciribine, TAT-Akt-in, Akt-l-1, 2, API-59CJ-OMe, Akt- inhibitor-X
BCL2- —> one that lowers expression of pro-apoptotic protein BCL2, Endostatin, catechin
IFNG- —> one that decreases cytokine IFNG amount, one that inhibit downstream signal JAK1, JAK2, STAT1, upadacitinib, peficitinib, tofacitinib, baricitinib, filgotinib, abrocitinib, delgocitinib
BATF3- → one that lowers expression of transcription factor BATF3, TGFβR inhibitor SB431542, YL-13027, galunisertib, SB-505124, SH3051, vactosertib, IN1233, PF-06952229, SJN2511, SB-525334, SM1-71, GFH018, AKT inhibitor (catechin, which stops the upstream of AKT, is effective)SH-5, Perifosine, Triciribine, TAT-Akt-in, Akt-l-1, 2, API-59CJ-OMe, Akt- inhibitor-X
CCL3- -> one that prevents signal transduction due to stimulation or inhibits signal transduction
BCL2L11-→ one that lowers expression of anti-apoptosis protein BCL2L11
BCL2L14-→ one that lowers expression of anti-apoptosis protein BCL2L14
BCL2A1- -> one that lowers expression of anti-apoptosis protein BCL2A1
BCL2L15-→ one that lowers expression of anti-apoptosis protein BCL2L15
MEK1- -> MEK1 inhibitor (PD0325901, Trametinib Dimethyl Sulfoxide, Binimetinib, Selumetinib Sulfate, binimetinib, vemurafenib, ARRY-424704, cobimetinib, encorafenib, dabrafenib, trametinib, dabrafenib, pembrolizumab, trametinib, TAK 733, binimetinib, encorafenib, panitumumab, E 6201, refametinib, PD184352, binimetinib, trametinib, dabrafenib, ipilimumab, trametinib, RO4927350, cobimetinib, atezolizumab, cobimetinib, vemurafenib, U0126, pimasertib, trametinib, vemurafenib, AS703988, dabrafenib, trametinib, vemurafenib, docetaxel, selumetinib, SM1-71, binimetinib, cetuximab, encorafenib, MKK1 inhibitor, mirdametinib, cetuximab, dabrafenib, trametinib, PD318088, cobimetinib, vemurafenib, cobimetinib, dabrafenib, binimetinib, dabrafenib, selumetinib, binimetinib, encorafenib, FCN-159, trametinib, vorinostat), PD98059, U0126, FR180204
MEK2- -> MEK2 inhibitor (PD0325901, Trametinib Dimethyl Sulfoxide, Binimetinib, Selumetinib Sulfate, binimetinib, vemurafenib, ARRY-424704, cobimetinib, encorafenib, dabrafenib, trametinib, dabrafenib, pembrolizumab, trametinib, TAK 733, binimetinib, encorafenib, panitumumab, E 6201, refametinib, PD184352, binimetinib, trametinib, dabrafenib, ipilimumab, trametinib, RO4927350, cobimetinib, atezolizumab, cobimetinib, vemurafenib, U0126, pimasertib, trametinib, vemurafenib, AS703988, dabrafenib, trametinib, vemurafenib, docetaxel, selumetinib, SM1-71, binimetinib, cetuximab, encorafenib, MKK1 inhibitor, mirdametinib, cetuximab, dabrafenib, trametinib, PD318088, cobimetinib, vemurafenib, cobimetinib, dabrafenib, binimetinib, dabrafenib, selumetinib, binimetinib, encorafenib, FCN-159, trametinib, vorinostat), PD98059, U0126, FR180204 GSK3β- → GSK3β inhibitor (catechin, which stops the upstream of GSK3β, is effective)LiCl, BIO.SB-216763, SB-415286, Quercetin, Ionomycin, ICG-001, PFK115-584, CPG049090, PNU-74654, CHIR99021
AKT- —> AKT inhibitor (catechin, which stops the upstream of AKT, is also effective)SH-5, Perifosine, Triciribine, TAT-Akt-in, Akt-l-1, 2, API-59CJ-OMe, Akt- inhibitor-X
mTOR- —> mTOR inhibitor ((Rapamycin, Everolimus, Temsirolimus, everolimus, fulvestrant, apitolisib, everolimus, pasireotide, corticosteroid, sirolimus, PF-4691502, PP-121, GNE-493AZD8055, dactolisib, PKI-179, onatasertib, samotolisib, SF2523, everolimus, letrozole, HEC68498, everolimus, prednisone, everolimus, gefitinib, PI-540, methotrexate, sirolimus, tacrolimus, prednisone, tacrolimus, PI-620, methylprednisolone, tacrolimus, cyclosporine A, sirolimus, tacrolimus, PWT33597, mTOR inhibitor, everolimus, panobinostat, everolimus, sorafenib, everolimus, vandetanib, ETP-45658, WXFL10030390, bimiralisib, pimecrolimus, panulisib, DS-7423, everolimus, exemestane, PKI-402, everolimus, ribociclib, gedatolisib, lenalidomide, temsirolimus, OSI-027, VS-5584, everolimus, tamoxifen, ridaforolimus, everolimus, lenvatinib, vistusertib, everolimus, paclitaxel, mTOR inhibitor, tyrosine kinase inhibitor, ME-344, tacrolimus, imatinib, sirolimus), SB431542, YL-13027, galunisertib, SB-505124, SH3051, vactosertib, IN1233, PF-06952229, SJN2511, SB-525334, SM1-71, GFH018, which stop TGFβR upstream of mTOR)FK506, NFAT inhibitor, Lerdelimumab, Metelimumab, GC-1008, LY550410, SB-505124, SD-208)
c-Myc- → c-Myc inhibitor Endostatin, TMPyP4
GLS1- → GLS1 inhibitor, KRAS, CB-839
PPAR- —> PPAR inhibitor (GW6471, Rosiglitazone, GW9662, catechin, which stops the upstream of PPAR, is also effective), Ro41-5253, guggulsterone
statin- —> HMG-CoA reductase inhibitor (simvastatin, Atorvastatin Calcium Hydrate, Rosuvastatin Calcium, Pitavastatin Calcium Hydrate, Fluvastatin Sodium, Pitavastatin Calcium, atorvastatin, choline fenofibrate, lovastatin, niacin, lovastatin, crilvastatin, aspirin, pravastatin, fluvastatin, atorvastatin, niacin, beta-hydroxy simvastatin acid, atorvastatin, ezetimibe, fluvastatin, fenofibrate, simvastatin, ezetimibe, rosuvastatin, atorvastatin, ezetimibe, simvastatin, sitagliptin, cerivastatin, pitavastatin, pravastatin, fenofibric acid, rosuvastatin, ezetimibe, simvastatin, amlodipine, ezetimibe, losartan, rosuvastatin, fenofibrate, pravastatin, amlodipine, atorvastatin)
PFK1- —> PFK1 inhibitor, Tryptolinamide
IRBIT- —> one that decreases signal transduction mediated by regulatory protein IRBIT
Wnt- -> Wnt inhibitor NSC668036, Imatinib, LiCl, BIO.SB-216763, SB-415286, Quercetin, Ionomycin, ICG-001, PFK115-584, CPG049090, PNU-74654, GSK3 inhibitor
p16- —> one that suppresses discontinuation of cell cycle by p16
p21- —> one that suppresses discontinuation of cell cycle by p21
CD28- —> one that prevents signal transduction due to stimulation or inhibits signal transduction
CD249- —> one that prevents signal transduction due to stimulation or inhibits signal transduction
CD150- —> one that prevents signal transduction due to stimulation or inhibits signal transduction
CD283- → one that prevents signal transduction due to stimulation or inhibits signal transduction
CD131- → one that prevents signal transduction due to stimulation or inhibits signal transduction
CD66a- —> one that prevents signal transduction due to stimulation or inhibits signal transduction
CD253- —> one that prevents signal transduction due to stimulation or inhibits signal transduction
CD120- —> one that prevents signal transduction due to stimulation or inhibits signal transduction
CD61- -> one that prevents signal transduction due to stimulation or inhibits signal transduction
CD87- -> one that prevents signal transduction due to stimulation or inhibits signal transduction
CD280- —> one that prevents signal transduction due to stimulation or inhibits signal transduction
CD195- —> one that prevents signal transduction due to stimulation or inhibits signal transduction
CD54- —> one that prevents signal transduction due to stimulation or inhibits signal transduction
CD265- —> one that prevents signal transduction due to stimulation or inhibits signal transduction
CD203a- —> one that prevents signal transduction due to stimulation or inhibits signal transduction
CD271- —> one that prevents signal transduction due to stimulation or inhibits signal transduction
CD68- —> one that prevents signal transduction due to stimulation or inhibits signal transduction
CD307c- —> one that prevents signal transduction due to stimulation or inhibits signal transduction
CD357- —> one that prevents signal transduction due to stimulation or inhibits signal transduction
CD196- —> one that prevents signal transduction due to stimulation or inhibits signal transduction
CD25- —> one that prevents signal transduction due to stimulation or inhibits signal transduction
CD154- → one that prevents signal transduction due to stimulation or inhibits signal transduction
CD203c- → one that prevents signal transduction due to stimulation or inhibits signal transduction
CD106- —> one that prevents signal transduction due to stimulation or inhibits signal transduction
CD244- —> one that prevents signal transduction due to stimulation or inhibits signal transduction
CD71- -> one that prevents signal transduction due to stimulation or inhibits signal transduction
CD220- —> one that prevents signal transduction due to stimulation or inhibits signal transduction
CD158e- -> one that prevents signal transduction due to stimulation or inhibits signal transduction
CD123- —> one that prevents signal transduction due to stimulation or inhibits signal transduction
CD16b- —> one that prevents signal transduction due to stimulation or inhibits signal transduction
CD364- —> one that prevents signal transduction due to stimulation or inhibits signal transduction
CD230- —> one that prevents signal transduction due to stimulation or inhibits signal transduction
CD41- —> one that prevents signal transduction due to stimulation or inhibits signal transduction
CD361- —> one that prevents signal transduction due to stimulation or inhibits signal transduction
CD161- —> one that prevents signal transduction due to stimulation or inhibits signal transduction
CCR4- —> one that prevents signal transduction due to stimulation or inhibits signal transduction
CD292- —> one that prevents signal transduction due to stimulation or inhibits signal transduction
IL-21- → IL-21

The activation treatment may be performed in any way as long as the desired effect can be obtained. For example, when the stimulating substance is an antibody, the activation treatment may be performed by adding an anti-CD62L antibody alone, an anti-CD327 antibody alone, or a combination of an anti-CD62L antibody and an anti-CD327 antibody to a medium and culturing same for 1 second to 20 days, preferably 1 second to 14 days, further preferably 1 second to 10 days to apply stimulation by the anti-CD62L antibody or anti-CD327 antibody. Also, stimulation by the anti-CD62L antibody or anti-CD327 antibody can be applied by culturing in a culture container (e.g., culture dish) with a culture surface coated with an anti-CD62L antibody alone, or an anti-CD327 antibody alone, or a combination of an anti-CD62L antibody and an anti-CD327 antibody, for 1 second to 20 days, preferably 1 second to 14 days, further preferably 1 second to 10 days. For example, when the stimulating substance is an inhibitor, Rapamycin alone, or simvastatin alone, or a combination of Rapamycin and simvastatin is added to a medium and the mixture is cultured for 1 second to 20 days, preferably 1 second to 14 days, further preferably 1 second to 10 days, to apply stimulation by Rapamycin or simvastatin.

### (Antigen recognition treatment of CAR-T cells after CAR gene introduction)

As a treatment after CAR gene introduction, an antigen recognition treatment is further performed to impart CAR-T cells with antitumor property. The method of the antigen recognition treatment is not particularly limited as long as the desired effect on CAR-T cells can be obtained. For example, it can be performed by contacting with a recognition substance which is an antigen to be the target of CAR. By performing an antigen recognition treatment, CAR-T cells can specifically recognize antigens. Furthermore, they become CAR-T cells that have antitumor property against the recognized antigen.

As the tumor-associated antigen or tumor specific antigen that is targeted by CAR as a recognition substance, for example, EPHA2, HER2, EPHB2, EPHB4, EGFR, GD2, Glypican-3, HER2, 5T4, 8H9, αvβ6 integrin, B cell mature antigen (BCMA), B7-H3, B7-H6, CAIX, CA9, CD19, CD20, CD22, κ light chain, CD30, CD33, CD38, CD44, CD44v6, CD44v7/8, CD70, CD116, CD123, CD138, CD171, CEA, CSPG4, EGFR, EGFRvIII, EGP2, EGP40, EPCAM, ERBB3, ERBB4, ErbB3/4, FAP, FAR, FBP, fetal AchR, folate receptor α, GD3, HLA-AI MAGE A1, HLA-A2, IL11Ra, IL13Ra2, KDR, lambda, Lewis Y, MCSP, mesoserine, MUC1, MUC4, MUC6, NCAM, NKG2D ligand NY-ESO-1, PRAME, PSCA, PSC1, PSMA, ROR1, Sp17, SURVIVIN, TAG72, TEM1, TEM8, VEGR receptor 2, cancer fetal antigen, HMW-MAA, VEGF receptor, fibronectin, tenascin, factors that enhance cancer migration (e.g., chemokines) or antigens present in the extracellular matrix, such as carcinoembryonic antigen (CEA) in necrotic areas of the tumor, or proteins containing mutations identified by genomic analysis and/or differential expression study of tumor, and the like can be mentioned. EPHB4, GD2, HER2, and CD19 are preferred, and EPHB4, HER2, and CD19 are more preferred.

Furthermore, a co-stimulating substance can be added in view of the maintenance/proliferation of T cells and/or CAR-T cells. The co-stimulating substance is not limited as long as it stimulates co-stimulation molecules in T cells or CAR-T cells, and includes CD80, CD86, 4-1BBL, OX40L, ICOS-L, CD70, CD40L, CD270, ICAM-1, LFA-3, CD72, CD55, VCAM-1, MadCAM-1, CD111, CD112, CD155, CD153, PD-L2, PD-L1, Galectin-9, MHC, CD113, and the like.

The tumor-associated antigen or tumor specific antigen that is targeted by CAR can be used as cells expressing them. That is, CAR-T cells can be expansion cultured by co-culturing them as feeder cells.

This feeder cell is a cell that has been engineered to express part or all of the target antigen on the cell surface so that the CAR introduced into the CAR-T cell can bind to the target antigen. The target antigen includes, for example, tumor-associated antigens or tumor-specific antigens that are targets of the aforementioned CAR. Feeder cells can be prepared by, as an operation to express a target antigen and as described above for CAR-T cells, introducing a gene encoding the target antigen into cells by using a vector having an expression unit for expressing the target antigen gene. Alternatively, feeder cells can also be prepared by producing mRNA of a target antigen gene and directly introducing the mRNA into cells.

In addition, the target antigen, a co-stimulating substance, and a stimulation factor may be expressed on the surface of feeder cells by an operation to introduce genes for co-stimulating substance and stimulation factor (here, stimulation factor (+)) into feeder cells together with the target antigen gene. That is, in a certain embodiment, the target antigen-expressing cell contains genes for one or more types of exogenous co-stimulating substances and stimulation factors.

As a method for introducing genes for co-stimulating substances and stimulation factors, a method of introducing genes by using an expression vector containing the genes of a co-stimulating substance and a stimulation factor, and the target antigen gene; and a method of introducing an expression vector or mRNA for a stimulation factor different from the expression vector or mRNA for the target antigen, simultaneously or separately from the expression vector or mRNA for the target antigen can be mentioned.

Expansion culture using feeder cells can be performed according to Patent Literature 1.

Another embodiment of the present invention is characterized in that the activation treatment of T cells before CAR gene introduction and the expansion culture after CAR gene introduction (including CAR-T cell activation treatment after CAR gene introduction and CAR-T cell antigen recognition treatment after CAR gene introduction) are performed in the absence of feeder cells. Activation treatment in the absence of feeder cells is, for example, contact with a stimulating substance (described above), and is specifically performed by contacting with a substrate to which a stimulating substance is bound, contacting with vesicles encapsulating a stimulating substance, or culturing in a medium containing a stimulating substance. In addition, antigen recognition treatment in the absence of a feeder cell is, for example, contact with a recognition substance (described above), and specifically performed by contacting with a substrate to which a recognition substance is bound, contacting with vesicles containing a recognition substance, or culturing in a medium containing a recognition substance. Furthermore, when a co-stimulating substance (described above) is used in expansion culture after CAR gene introduction, it is performed by contacting with a substrate to which a co-stimulating substance is bound, contacting with a vesicle containing a co-stimulating substance, or culturing in a medium containing a stimulating substance.

The combinations of the activation treatment, antigen recognition treatment, and treatment with a co-stimulating substance of T cells in the absence of feeder cells are not particularly limited as to the embodiments thereof as long as the desired effect is obtained. For example, various treatments of T cells in the absence of feeder cells can be performed using the following combinations, and are not limited to these:
(Embodiment 1)
   added to medium: stimulating substance, recognition substance, co-stimulating substance
   bound to substrate and/or encapsulated in vesicle: none
(Embodiment 2)
   added to medium: stimulating substance, recognition substance bound to substrate and/or encapsulated in vesicle: co-stimulating substance
(Embodiment 3)
   added to medium: stimulating substance, co-stimulating substance
   bound to substrate and/or encapsulated in vesicle: recognition substance
(Embodiment 4)
   added to medium: recognition substance, co-stimulating substance
   bound to substrate and/or encapsulated in vesicle: stimulating substance
(Embodiment 5)
   added to medium: stimulating substance
   bound to substrate and/or encapsulated in vesicle: recognition substance, co-stimulating substance
(Embodiment 6)
   added to medium: recognition substance
   bound to substrate and/or encapsulated in vesicle: stimulating substance, co-stimulating substance
(Embodiment 7)
   added to medium: co-stimulating substance
   bound to substrate and/or encapsulated in vesicle: stimulating substance, recognition substance
(Embodiment 8)
   added to medium: none
   bound to substrate and/or encapsulated in vesicle: stimulating substance, recognition substance, co-stimulating substance

Although not shown in the aforementioned examples, an embodiment may be adopted in which, for example, a stimulating substance is added to the medium and simultaneously bound to the substrate. When such an embodiment is adopted, for example, the stimulating substance to be added to the medium and the stimulating substance to be bound to the substrate may be the same or different.

In addition, only one kind or multiple kinds of substances may be added to the medium, bound to the substrate, and encapsulated in the vesicles. For example, when a co-stimulating substance is to be bound to a substrate, only one kind or two or more kinds of co-stimulating substances may be bound.

Substrates to which a stimulating substance, a recognition substance, and/or a co-stimulating substance (hereinafter sometimes referred to as "stimulating substance, etc.") are to be bound may be those in the form of film, sponge, fiber, rod, bead, or gel. Particularly preferred is a bead (particle size of 10 nm to 500 µm, preferably 10 nm to 100 µm, more preferably 10 nm to 50 µm) or a gel. As the materials, magnetic substance, latex, agarose, glass, cellulose, sepharose, nitrocellulose, polystyrene, retronectin, collagen, and the like can be mentioned.

Examples of vesicles encapsulating stimulating substance, etc. include liposomes, exosomes, microvesicles, and apoptotic bodies.

Contact with the stimulating substance, etc. is preferably performed by culturing in a medium containing stimulating substance, etc. (e.g., including stimulating substance, recognition substance, or co-stimulating substance bound to substrate, stimulating substance, recognition substance, or co-stimulating substance encapsulated in vesicle), namely, co-culturing CAR-T cells with stimulating substance, etc.

### Co-culture

By co-culturing CAR-T cells with a stimulating substance, etc., CAR-T cells proliferate efficiently by stimulation with the stimulating substance, etc.

For cell recovery and stable expression of the introduced gene after CAR gene introduction, CAR-T cells are preferably cells that have been cultured for, for example, about 1 second to 2 weeks. Since CAR-T cells may be exhausted by long-term culture, they are more preferably subjected to co-culture within 1 second to 1 week, within 1 second to 72 hours, or within 1 second to 48 hours, after CAR gene introduction.

While the co-culture period is not limited, it is 1 second to 21 days, preferably 1 second to 14 days.

When target antigen-expressing cells (feeder cells) are used as recognition substances, the ratio of CAR-T cells to feeder cells (CAR-T cells/feeder cells) at the start of co-culture is not particularly limited. For example, the ratio of the cells that have been manipulated to express CAR or the target antigen to the total number of cells is 0.05 to 20, preferably 0.1 to 10, more preferably 0.5 to 5, respectively. The cell density during co-culture is, for example, 1×10⁶ cells/mL to 100×10⁶ cells/mL, as the concentration of cells in the culture medium.

When the activation treatment, antigen recognition treatment and/or treatment with a co-stimulating substance are/is performed in the absence of a feeder cell, the stimulating substance, etc. are subjected to each treatment in a state of being bound to the substrate, being encapsulated in vesicles, or being contained in the medium. The dose of the stimulating substance, etc. at that time is not particularly limited as long as it is an amount that permits sufficient activation and proliferation of CAR-T cells, and is appropriately determined according to the kind of the stimulating substance, etc. Generally, an excess amount is added and used.

The medium used during co-culture and when preparing CAR-T cells and/or feeder cells is not particularly limited, and a medium used for normal cell culture, such as RPMI1640, MEM, X-VIVIO, IMDM, DMEM, DC medium, OptiMEM, and the like can be used. The medium may be a medium to which serum (human serum, fetal bovine serum, etc.) is added according to a conventional method, or a serum-free medium. It is preferable to use a serum-free medium because it is highly safe for clinical application and the culture efficiency is less likely to vary due to differences between serum lots. Examples of serum-free media include TexMACS TM (Miltenyi Biotec), AIM V (registered trademark) (Thermo Fisher Scientific), ALyS culture medium (Cell Science & Technology Institute, Inc.), and the like. When using serum, autologous serum, that is, serum collected from the individual from whom the CAR-expressing immune cells are derived (more specifically, a patient receiving administration of the cell population obtained by the production method of the present disclosure) may also be used, or it may also be an artificial serum. In the present invention, it is preferable to use artificial serum. Plasma may also be used. Blood components such as serum or plasma, albumin, etc. or analogs thereof may be used, and artificial substances may also be used. As the basal medium, one suitable for cell culture may be used, and the aforementioned TexMACS TM (Miltenyi Biotec), AIM V (registered trademark), and ALyS culture medium (Cell Science & Technology Institute, Inc.) can be used. Other culture conditions may be those suitable for cell survival and proliferation, and general conditions may be employed. For example, culturing in a CO₂ incubator (CO₂ concentration 5%) set at 37°C, hypoxic culture (oxygen concentration 0 to 20%, preferably 0 to 10%, more preferably 1 to 5%), and the like can be mentioned.

Additional factors may be added to the medium in order to support cell survival and proliferation. As the additional factor, type 1 cytokine family member, type 2 cytokine family member, TNF superfamily cytokine, IL-1 family cytokine, other cytokines (TNF-β, etc.) can be mentioned, and specifically IL-1 to IL-41 and the like, preferably IL-1, IL-2, IL-7, IL-15, IL-21, and the like, can be mentioned. When preparing CAR-T cells, IL-7 and/or IL-15 may be added to the medium. Additional factors can be prepared according to conventional methods, and commercially available products can also be used. Additional factors may be from an animal species other than humans, but are preferably derived from humans (and may be recombinant).

By expansion culturing CAR-T cells (co-culturing with stimulating substance, recognition substance and/or co-stimulating substance), a cell population containing CAR-T cells can be obtained in sufficient quantity and quality for clinical use. The method of the present invention can efficiently produce a CAR-T cell population that is expected to be highly effective compared to conventional methods. For example, the CAR-T cell population obtained by the method of the present disclosure may have a proportion of CAR-T cells of 20%, 30%, or 40% or more, preferably 40% or more.

When T cells continue to be chronically activated, they highly express a large number of immune checkpoint molecules, and become unable to proliferate or attack target cells. This phenomenon is called exhaustion, and exhausted T cells have weak ability to proliferate and attack cancer even when returned to the body, and cannot be expected to show a high therapeutic effect. In the cell population obtained by the method of the present invention, exhaustion is suppressed. The degree of exhaustion can be evaluated by methods practiced in the art. For example, it can be evaluated by examining the expression status of exhaustion markers, which is also convenient. Exhaustion markers include Programmed death 1 (PD-1), T-cell immunoglobulin mucin-3 (Tim-3), lymphocyte activation gene 3 (LAG3), Adenosine A2a receptor (A2aR), Cytotoxic T-lymphocyte-associated antigen 4 (CTLA4), T cell immuno receptor with Ig and ITIM domains TIGIT, and the like, and particularly PD-1 can be mentioned. Expression of exhaustion markers can be detected using antibodies against these markers. The CAR-T cell population obtained by the method of the present disclosure has low expression of the exhaustion marker PD-1. For example, the proportion of PD-1 expressing cells among CAR-T cells is less than 10%, preferably less than 5%, more preferably less than 1%.

Furthermore, the CAR-T cell population obtained by the methods of the present disclosure may have a proportion of naive or young memory T cells in the CAR-T cells of 20%, 30%, 45%, 50%, 55%, or 60% or more, preferably 60% or more.

After expansion culture, CAR-T cells are collected. The collection operation may be performed in a conventional manner. For example, the cells are collected by pipetting, using a liquid feeding tube, centrifugation treatment, or the like. In a preferred embodiment, before the collection operation, a step of culturing the cells after expansion culture in the presence of a stimulating substance is performed. This step enables efficient expansion culture and also has the advantage of increasing cell survival rate. When desired, the CAR-T cell population after expansion culture may be subjected to a cell separation step such as bead separation. By performing the cell separation step, the purity of CAR-T cells with higher effect can be increased,

A cell population containing CAR-T cells produced by the method of the present invention can be used for the treatment of cancer, particularly for the treatment of cancer that expresses the target antigen of the CAR-expressing immune cells. The cancer may be a solid tumor or a hematologic tumor. Specific examples of the cancer include, but are not limited to, various B-cell lymphoma (follicular malignant lymphoma, diffuse large B-cell malignant lymphoma, mantle cell lymphoma, MALT lymphoma, intravascular B-cell lymphoma, CD20-positive Hodgkin lymphoma, and the like), bone marrow proliferative tumor, bone marrow dysplasia/bone marrow proliferative tumor (CMML,JMML,CML,MDS/MPN-UC), myelodysplastic syndrome, acute myeloid leukemia, neuroblastoma, brain tumor, Ewing's sarcoma, osteosarcoma, retinoblastoma, small cell lung cancer, non-small cell lung cancer, melanoma, bonesoft tissue sarcoma, kidney cancer, pancreatic cancer, malignant mesothelioma, prostate cancer, breast cancer, endometrial cancer, cervical cancer, ovarian cancer, colorectal cancer, and the like. In preferred embodiments, the cancer is a solid tumor. Examples of the solid tumor include neuroblastoma, brain tumor, Ewing's sarcoma, osteosarcoma, retinoblastoma, small cell lung cancer, non-small cell lung cancer, melanoma, ovarian cancer, rhabdomyosarcoma, bonesoft tissue sarcoma, kidney cancer, pancreatic cancer, malignant mesothelioma, prostate cancer, breast cancer, endometrial cancer, cervical cancer, ovarian cancer, colorectal cancer, and the like.

A cell population containing CAR-T cells produced by the method of the present invention is administered in a therapeutically effective amount that is appropriately determined according to the age, weight, body surface area, symptoms, and the like of the subject. The subject in the present disclosure is generally a human, preferably a cancer patient. A cell population containing CAR-T cells produced by the method of the present invention can be administered at, for example, 1×10⁴ cells to 1×10¹⁰ cells per dose. The route of administration is not particularly limited, and the cells can be administered intratumorally, peritumorally, intraventricularly, intravenously, intraarterially, intraportally, intradermally, subcutaneously, intramuscularly, or intraperitoneally. The cell population of the present disclosure may be administered systemically or locally, and local administration includes direct injection into the target tissue, internal organ, or organ. The administration schedule is appropriately determined according to the age, body weight, body surface area, symptom, and the like of the subject, and may be a single administration or continuous or periodic multiple administrations.

A composition containing a cell population containing CAR-T cells produced by the method of the present invention may contain, in addition to the cell population to be administered to a subject, components such as dimethyl sulfoxide (DMSO), serum albumin, and the like for the purpose of cell protection, antibiotics and the like for the purpose of preventing bacterial contamination, various components (vitamins, cytokine, mineral, carbon source, nitrogen source, trace metal, electrolyte, growth factor, steroid, etc.) for the purpose of cell activation, proliferation, or differentiation induction, and the like. The composition can be prepared by conventional methods.

The present invention is described in detail below using examples, but the present invention is not limited in any way. Reagents and materials to be used are commercially available unless otherwise specified, or can be prepared from known literature and the like. In addition, those skilled in the art understand that other substances having similar effects and actions can be used alternatively.

### [Example]

### Material and method

### 1. Ethical approval and consent to participation

This study was approved by the Institutional review committee of Kyoto Prefectural University of Medicine, and the recombinant DNA experiment was approved by the Recombinant DNA Experiment Safety Committee of Kyoto Prefectural University of Medicine. All experiments involving human participants were performed according to the guidelines of the Declaration of Helsinki. All animal experiments and procedures were approved by the Institutional review committee of Kyoto Prefectural University of Medicine.

### 2. Blood donor and cell line

Density gradient centrifugation was performed using lymphocyte separation medium 1077 (FUJIFILM Wako Pure Chemical Corporation, Osaka), and PBMC was separated from the whole blood sample by washing many times with Dulbecco phosphate buffered saline (D-PBS, Nacalai Tesque, Kyoto). The number of viable cells was measured using standard trypan blue staining and automatic Cell Counter model R1 (OLYMPUS CORPORATION, Tokyo, Japan). Human lymphoblastic leukemia cell line (REH) was purchased from American Type Culture Collection (Manassas, VA). REH (REH-FFLuc-GFP) expressing firefly luciferase (FFLuc) and green fluorescent protein (GFP) was obtained by introducing piggyBac transposon (PB)-based pIRII-FFLuc-puroR-GFP (Non Patent Literature 6) into REH cells, followed by fluorescenceactivated cell sorting (FACS). REH cells and REH-FFLuc-GFP cells were cultured in Roswell Park Memorial Institute-1640 (RPMI-1640) medium (Nacalai Tesque) supplemented with 10% fetal bovine serum (Thermo Fisher Scientific, Inc. Waltham, MA) and maintained under 5% CO₂ atmosphere in a humidified incubator at 37°C.

### 3. Production of CAR-T cells by PB method

CD45RA+ and CD45RA- PBMCs were separated from all PBMCs by magnetic separation using CD45RA MicroBeads, human (Miltenyi Biotec, Bergisch Gladbach, Germany) (Fig. 1). Then, CD19-CAR transgene was introduced into these cells by using a PB transposon system, as described in previous reports (Non Patent Literatures 5 and 6). In brief, PB transposase plasmid (7.5 µg per 100 µL of electroporation buffer) and pIRII-CD19-28z (7.5 µg/100 µL) (Fig. 2) were introduced into about 4×10⁶ CD45RA+ PBMC and CD45RA- PBMC, respectively, by using P3 Primary Cell 4D-Nucleofector^{™} X kit (Lonza, Program; FI-115) or MaxCyte ATX (registered trade mark) (MaxCyte Inc) and a protocol optimized for introduction of DNA plasmid into resting T cells (Protocol; RTC 14-3). Simultaneously, antigen-presenting feeder plasmid (pIRII-tCD19-CD80-41BBL; 15 µg per 100 µL) (Fig. 2) was introduced into about 1×10⁶ whole PBMC by electroporation. After the electroporation, CAR-T cells and feeder cells were cultured in a complete culture medium obtained by adding 5% artificial serum (Animal-free; Cell Science & Technology Institute), IL-7 (10 ng/mL; Miltenyi Biotec), and IL-15 (5 ng/mL; Miltenyi Biotec) to ALyS^{™}705 Medium (Cell Science & Technology Institute). The feeder cells were inactivated by UV irradiation 24 hr after the electroporation, and co-cultured with CAR-T cells for 14 days (Non Patent Literatures 5 and 6). CAR-T cells redirected by EPHB4 receptor were produced by a PB transposon system as described above, for use as control CAR-T cells in an in vivo stress test (Non Patent Literature 5) (Fig. 2) .

### 4. Flow cytometry

The expression of CD19-CAR molecule on T cell surface was measured by flow cytometry using a goat anti-human immunoglobulin (Ig)-G Fc fragment-specific antibody (Merck Millipore, Burlington, MA) to which recombinant human CD19 Fc chimera protein (R&D Systems, Minneapolis, MN, USA) and fluorescein isothiocyanate (FITC) were bound. In order to characterize the phenotype of CAR-T cells, allophycocyanin (APC)-conjugated anti-CD3 antibody, APC-conjugated anti-CD8 antibody, phycoerythrin (PE)-conjugated anti-CD4 antibody, PEconjugated anti-CD45RA antibody, and APC-conjugated anti-CCR7 antibody (all BioLegend, San Diego, CA, USA) were used. As exhaustion and aging markers for CAR-T cells, APC-labeled antiprogrammed cell death protein-1 (PD-1) antibody, APC-labeled anti-T cell immunoglobulin · mucin-3 (TIM-3) antibody, Alexa Fluor 647-labeled anti-CD223 (LAG-3) antibody, and Peridinin-Chlorophyll-Protein (PerCP)/Cyanine5.5-labeled anti-CD57 antibody were used (all manufactured by BioLegend). The phenotype of REH cell was determined using FITC-labeled anti-CD19 antibody (BioLegend). All flow cytometry data were obtained using BD Accuri^{™} C6 Plus or BD FACSCalibur^{™} (BD Biosciences, Franklin Lakes, NJ), and analyzed using FlowJo^{™} software (BD Biosciences).

### 5. Analysis of exhaustion marker expressed on T cell surface after electroporation

Electroporation was performed with the same protocol as that when production of CAR-T, and GFP plasmids were introduced into all PBMCs. After the electroporation, PBMC introduced with GFP was cultured in a complete culture medium composed of the same components as those used for production of CAR-T. PBMC free of electroporation was cultured in the same medium and used as a control. After culture for 48 hr, GFP-positive T cells and CD3-positive T cells were gated, and the expression of PD-1, TIM-3, and LAG-3 on the T cell surface was analyzed by flow cytometry.

### 6. Continuous killing assay

1×10⁵ REH cells and 1×10⁵ CD19 CAR-T cells derived from CD45RA+ PBMC or CD45RA- PBMC (RA+ CAR or RA- CAR, respectively) were co-cultured on a 24-well cell culture plate. After 3 days, CD19 CAR-T cells were collected, counted, treated with fresh REH cells at a ratio of 1:1, and reconstituted. The counting of cells and treatment with fresh REH cells were repeated every 3 days three times in total. The killing effect of these CD19 CAR-T cells was evaluated by counting the remaining REH cells by flow cytometry, and the CAR mean fluorescent intensity (MFI) and the exhaustion marker of these CAR-T cells were analyzed by flow cytometry.

### 7. RNA sequencing and bioinformatics analysis

RA+ CAR-T cells and RA- CAR-T cells were co-cultured with REH cells for 3 days such that the ratio of effector:target was 1:1 irrespective of the presence or absence of antigen stimulation (RA+/Stimulation+, RA-/Stimulation+, RA+/Stimulation-, RA-/Stimulation-, respectively), and total RNAs were isolated using RNeasy Mini Kit (Qiagen, Venlo, Netherlands). The concentration of total RNAs was measured using NanoDrop 2000 (Thermo Fisher Scientific). Library preparation and high-throughput sequencing were performed using Eurofins Genomics (Ebersberg, Germany). In brief, mRNA was concentrated and the strand-specific library thereof was prepared. Sequencing was performed using NextSeq 500/550 system (Illumina, San Diego, CA, USA) and NextSeq 500/550 Mid Output Kit v2.5 150 cycle (Illumina). Adapter sequences and low quality reads were removed using fastp version 0.21.0. The filtered reads were aligned to the human reference genome (GRCh38.p13) by using STAR version 2.7.6A. Using RSEM version 1.3.3, the count number per one gene and transcription products per million were calculated. The calculation of the counts per million and differential expression analysis were performed using edge R version 3.32.0 R package and R v4.0.3 environment (https://www.R-project.org/). Pathway analysis was performed using R package for the Reactome Pathway analysis). Furthermore, a differential gene expression profile between RA+ CAR and RA- CAR was analyzed and visualized using Morpheus (https://software.broadinstitute.org/morpheus) and specific gene signatures associated with activation, exhaustion, and differentiation of T cells.

### 8. In vivo stress test using systemic tumor model mouse

Female 8-week-old NOD.Cg-Prkdc^{scid}II2rg^{tm1Wjl}/SzJ(NSG) mice were purchased from Jackson Laboratory (Bar Harbor, ME, USA), and bred for more than one week at Kyoto Prefectural University of Medicine before the start of the experiment. The mice were allowed to freely take food and water. REH-FFLuc-GFP cells (5×10⁵) were suspended in D-PBS and injected into the mice via the tail vein. After 6 days, 1×10⁵ RA+ CAR-T cells, RA- CAR-T cells, or unrelated CAR-T cells redirected with EPHB4 receptor as a control were injected via the tail vein, and the tumor amount was monitored using IVIS Lumina Series III system (PerkinElmer, Inc). The region of interest displayed on the image was quantified as photons per second (ph/s) using Living Image v2 (PerkinElmer, Inc.) according to a previous report (Non Patent Literature 5). Bone marrow (BM) cells were obtained by continuous aspiration from the tibia at several time points. These BM cells were stained with PE-labeled anti-human CD3 antibody and APC-labeled anti-human PD-1 antibody (BioLegend), and the long-term sustainability of human T cells was evaluated by flow cytometry.

### 9. Statistics

For statistical comparison between two groups, unpaired data were determined by a two-tailed parametric test or a nonparametric test (Mann-Whitney U-test), and matched samples were determined by a two-tailed paired Student's t-test. All data are shown as mean±standard deviation. For comparison of the survival curves obtained by the Kaplan-Meier method, a log-rank test was used. A P value of less than 0.05 was judged to be statistically significant. All statistical analyses were performed using GraphPad Prism 9 software.

### Results

### Example 1

Using a magnetic separation method targeting human CD45RA, a subpopulation of CD45RA+ or CD45RA- was separated from whole PBMCs. As a result, two peaks of CD45RA high and negative were observed in the lymphocyte fraction, and CD45RA positive was evenly distributed from high to dim in the monocyte fraction (Fig. 1). By magnetic bead sorting, CD45RA+ and CD45RA- PBMCs were efficiently separated, and not less than 98% of CD45RApositive cells were separated in the CD45RA+ fraction and not less than 93% of CD45RA-negative cells were separated in the CD45RA- fraction (Fig. 1). Then, 24 hr after electroporation, a transient introduction efficiency of CD19 CAR transgene into CD45RA+ or CD45RA- PBMC was evaluated. When CD19 CAR transgene was introduced by electroporation into CD45RA+ or CD45RA- PBMC which was magnetically separated without stimulation, the CD45RA+ PBMC subpopulation showed higher transfection efficiency than the CD45RA- PBMC subpopulation 24 hr after electroporation (CD45RA+ PBMC:77.5%±9.8% vs. CD45RA-PBMC:39.7%±3.8%) (Fig. 3A). Furthermore, CD45RA+ PBMC-derived CD19 CAR-T cells (RA+ CAR-T) showed high amplification ability after 14 days of culture as compared with CD45RA- PBMC-derived CD19 CAR-T cells (RA- CAR-T) (RA+ CAR-T: 32.5±9.3-fold vs. RA-CAR-T:11.0±5.4-fold) (Fig. 3B). After 14 days of proliferation, positive degree of CAR in these CAR-T cells, phenotype, and expression of exhaustion marker PD-1 were measured by flow cytometry. In RA+ CAR-T cells, as compared with RA- CAR-T, high CAR-positive rate (RA+ CAR-T:68.3% ± 3.8% vs. RA- CAR-TES. 6% ± 7.3%), superior CD8 expression (RA+ CAR-T: 84.0% ±3.4% vs. RA- CAR-T: 34.1% ± 10.6%), low expression of exhaustion marker PD-1 (RA+ CAR-T:3.1% ± 2.5% vs. RA- CAR-T: 19. 2% ± 6.4%), decreased expression of T cell aging marker CD57 (RA+ CAR-T:6.8% ± 3.6% vs. RA- CAR-T: 20.2% ± 6.9%), naive/stem cell memory fraction (CD45RA+/CCR7+ fraction; RA+ CAR-T: 71.9% ± 9.7% vs. RA- CAR-T: 8.0% ± 5.3%), and the like were associated with the lifespan of CAR-T cell (Fig. 3C, Fig. 3D).

On the other hand, other activation/exhaustion markers such as TIM-3, LAG-3, and the like were highly expressed in both types of CAR-T cells (Fig. 3D), and this finding was consistent with previous studies relating to PB-CAR-T cells. Since most PB-CAR-T cells have been produced by electroporation to date, it was considered that the high expression of TIM-3 and LAG-3 might be induced by stimulation of electroporation. However, PBMC 48 hr after electroporation hardly expressed PD-1, TIM-3, and LAG-3 of T cell (data not shown). Therefore, it is considered that the expression of TIM-3 and LAG-3, rather than PD-1, on CAR-T cells was induced during the culture period, not by stimulation of electroporation.

### Example 2

The effect of RA+ CAR-T cells or RA- CAR-T cells was investigated at the molecular level, and genome-wide transcriptional profiling was performed by focusing on immunogenic gene signatures in order to identify pathways involved in the good phenotype of RA+ CAR-T cells. As compared with CD45RA- CAR-T cells, RA+ CAR-T cells were confirmed to show high expression of total 29 genes and low expression of 78 genes (Fig. 4A). As a result of reactome pathway analysis, the difference in the gene expression observed in RA+ CAR-T cells was associated with the co-stimulation by the non-canonical NF-κB pathway and the CD28 family pathway, and the PD-1 signal pathway was significantly downregulated in RA+ CAR-T cells (Fig. 4B). Transcriptome profiling also showed an activated but not exhausted profile of RA+ CAR-T cells, which is characterized by upregulation of T cell activation markers such as transcription factor 7, lymphocyte enhancer binding factor 1, CCR7.R, IL7R, and the like. In addition, RA+ CAR-T cells showed an activated but not exhausted profile including decreased expression of T cell activation markers such as PD-1, LAG3, T-cell immunoreceptor with Ig and ITIM domains (TIGIT), eomesodermin, and the like, even after antigen stimulation (data not shown). These results suggest that RA+ CAR-T cells have a rich naive/memory phenotype and are resistant to T cell exhaustion caused by antigen stimulation.

### Example 3

To evaluate the anti-leukemia activity of RA+ CAR-T and RA- CAR-T cells, fresh REH cells were added to CAR-T cells every 3 days, and a tumor rechallenge assay was performed. As a result, both of two types of RA+ CAR-T and RA- CAR-T cells could completely kill REH cells even after plural tumor rechallenges (Fig. 5A). The expression of CAR molecule on cell surface after antigen stimulation of RA+ CAR-T cells was controlled to relatively a lower level than that of RA- CAR-T cells (Fig. 5B). The expression of PD-1 in RA+ CAR-T cells was lower than that in RA-CAR-T cells during plural antigen stimulations (Fig. 5C). On the other hand, the expression of LAG-3 was similar in RA+ CAR-T cells and RA- CAR-T cells, but the expression of TIM-3 was higher in RA+ CAR-T cells than in RA- CAR-T cells. These expressions gradually decreased during plural antigen stimulations in RA+ CAR-T cells and RA- CAR-T cells, and comparatively high expressions of TIM-3 and LAG-3 did not impair the killing effect of CAR-T cells (Fig. 5C).

### Example 4

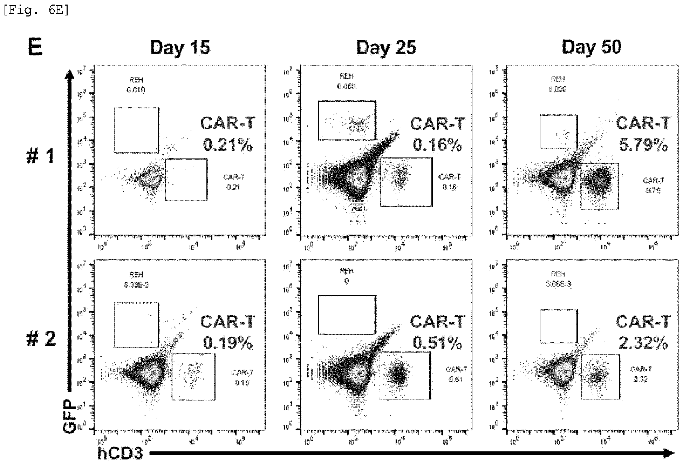

To evaluate the in vivo antitumor effects of RA+ CAR-T cells and RA- CAR-T cells, an in vivo stress test was performed in which the dose of CAR-T cells was lowered to the functional limit thereof and these CAR-T cells were maintained and proliferated in vivo to achieve antitumor effects. 5×10⁵ REH-FFLuc cells were injected to NSG mice via the tail vein. After 6 days, 1×10⁵ RA+ CAR-T, RA- CAR-T, or control (EPHB4) CAR-T-positive cells were injected to each mouse via the tail vein. RA+ CAR-T cells induced greater tumor shrinkage than RA- CAR-T cells and prolonged the median survival time (Figs. 6A to C). On day 15, in the bone marrow of the RA+ CAR-T cell group, human CD3-positive T cells with low PD-1 expression were abundant as compared with the RA- CAR-T cell group, and the number of REH cells was also comparatively small (Fig. 6D). Furthermore, continuous bone marrow tests confirmed that human CD3-positive T cells proliferated even 50 days after administration in two of the long-lived mice in the RA+ CAR-T group (Fig. 6E). This indicates that RA+ CAR-T cells proliferate in vivo by antigens and function over a long period.

### Example 5

Expansion culture of CAR-T cells was performed in the presence or absence of feeder cells and the effect thereof was examined. In this Example, a mixture of three kinds of stimulation beads was used, but the Example can also be performed with one or two kinds thereof. Four or more kinds thereof can also be used. When two or more kinds of stimulation beads are used, the combination thereof is not particularly limited.

### (Material and method)

### 1. Protein reconstitution

(i) CD19 protein (Aero BIOSYSTEMS)
(ii) CD80 protein (Aero BIOSYSTEMS)
(iii) 4-1BBL protein (Aero BIOSYSTEMS)

### 2. Stimulation bead preparation

Miltenyi MACSiBeads are each reacted with the above-mentioned 1 and used for CAR-T stimulation.

### 3. Production of CAR-T cells

### Day 0

Blood was collected, and CD19CAR/PB transposase and antigen-presenting feeder (AP feeder) were each subjected to electroporation (EP).

Culture was started under the following 4 conditions
1) AP
2) Beads 2.5M
3) Beads 5M
4) Beads 10M

### Day 1

A part of AP feeder is irradiated with UV and added to 1). 2.5M, 5M, and 10M of beads are respectively added to 2) to 4).

### Day 3

Expansion culture is performed for each.

### Day 8

Cell counting and CAR expression rate measurement
The remaining cells are split into two.
1) AP
2) AP (same as 1)
3) Beads 2.5M
4) Beads 2.5M+2.5M
5) Beads 5M
6) Beads 5M+5M
7) Beads 10M
8) Beads 10M+10M
2.5M, 5M, and 10M of beads are respectively added again to 4), 6), and 8). A culture medium is added and culture is further continued.

### Day 13

Cells were counted and CAR expression rate was measured and graphed.

The results are shown in Fig. 7. A higher proliferation rate was observed when expansion culture was performed using beads bound with stimulation factors than when expansion culture was performed using feeder cells.

### Example 6

From the above-mentioned Examples, it was found that CAR-T cells produced from CD45RA+ cells have good gene transfer efficiency, high cell proliferation potency, high anti-cancer effect, and low exhaustion marker expression rate. Thus, a candidate for specific factor other than CD45RA+ was investigated.

Omics analysis was performed in four kinds: "CD45RA+ with or without stimulation" and "CD45RA- with or without stimulation". As a result of the analysis, several specific factors were found (see Table 1).

### Example 7

EPHB4-CAR-T cells were produced from cryopreserved PBMC. Frozen PBMC (CTL) was thawed by allowing to stand in a 37°C water bath, diluted with Dulbecco's phosphate buffered saline (D-PBS, Wako), and then the number of cells was measured using NC-3000 (M&S TechnoSystems, Inc.).

The thawed PBMCs were centrifuged, and the cell pellets were suspended in electroporation buffer (Miltenyi Biotec). PB transposase plasmid and pIRII-EPHB4-28z were added to the obtained cell suspension and introduced into PBMC by electroporation.

After gene transfer, the cells were transferred to a complete culture medium obtained by adding 5% artificial serum (Animal-free; Cell Science & Technology Institute), IL-7 (10 ng/mL; Miltenyi Biotec), and IL-15 (5 ng/mL; Miltenyi Biotec) to ALySTM705 Medium (Cell Science & Technology Institute) and cultured for 14 days. At that time, beads to which a recognition substance and a co-stimulating substance (EPHB4-CD80-4-1BBL) were bound were added on day 1 and day 8 after electroporation, and the cells were expansion cultured.

The CAR-T cells obtained after culturing were subjected to cell count measurement, FCM analysis, and anticancer tests.

Also in the method of producing CAR-T cells from frozen PBMC, the cell proliferation ability was high as in the case of using fresh blood (13.8-fold proliferation ability), and the CAR-positive rate was at a high level of 74.7%. In the obtained CAR-T cells, the percentage of naive and stem cell memory T cells (Tscm) was 25% according to the results of FCM analysis, the exhaustion marker PD-1 was hardly expressed, and a high anti-cancer effect was also confirmed. From the above results, CAR-T cells could be produced also from frozen PBMC in the same way as from fresh blood. Similar results were obtained even when CD19 was used as the CAR gene (data not shown).

### Example 8

A comparison was made between flow cytometry method and bead separation method as methods for separating T cell subpopulations.

PBMC was separated from the whole blood sample by performing a density gradient centrifugation using lymphocyte separation medium 1077 (FUJIFILM Wako Pure Chemical Corporation), and washing many times with Dulbecco phosphate buffered saline (Nacalai Tesque). CD45RA+ and CD45RA- PBMCs were separated from all PBMCs by magnetic separation using CD45RA MicroBeads, human (Miltenyi Biotec, Bergisch Gladbach, Germany).

When separation from the whole PBMCs is performed by flow cytometry, whole PBMCs were stained with anti-CD45RA antibody and separated using a cell sorter.

The time required for the flow cytometry method and the bead separation method depending on the amount of the collected cells is summarized in Table 4.

**[Table 4]**

| amount of collected CD45RA-positive cells [cells] | magnetic bead method [hr] | flow cytometry method [hr] |
|---|---|---|
| 4.00 × 10⁶ | 0.5 | 2 |
| 1.00 × 10⁷ | 0.5 | 5 |
| 1.00 × 10⁸ | 0.5 | 50 |
| 1.00 × 10⁹ | 0.5 | 500 |
| 1.00 × 10¹⁰ | 0.5 | 5000 |

When compared with the flow cytometry method, the bead separation method can be performed in a closed system, and T cell subpopulations can be easily obtained in a short time regardless of the amount of the collected cells. From the above results, it is preferable to use a bead separation method that places less burden on cells and is available for clinical applications.

### Example 9

Separation of T cell subpopulation by using a specific factor was verified.

Frozen PBMC (CTL) was thawed by allowing to stand in a 37°C water bath, diluted with Dulbecco's phosphate buffered saline (D-PBS, Wako), and then the number of cells was measured using NC-3000 (M&S TechnoSystems, Inc.).

The thawed PBMCs were centrifuged, and the cell pellets were suspended in MACS buffer (0.5% BSA, 2 mM EDTA in D-PBS), and cell (CD45RA+) with CD45RA expressed on the cell membrane surface were separated using CD45RA MicroBeads, human (Miltenyi Biotec, Bergisch Gladbach, Germany).

After separation, the number of cells was measured, the required amount of cell suspension was centrifuged, and the cell pellets were suspended in electroporation buffer. PB transposase plasmid and pIRII-EPHB4-28z were added to the obtained cell suspension and introduced by electroporation.

After gene transfer, the cells were transferred to a complete culture medium obtained by adding 5% artificial serum (Animal-free; Cell Science & Technology Institute), IL-7 (10 ng/mL; Miltenyi Biotec), and IL-15 (5 ng/mL; Miltenyi Biotec) to ALySTM705 Medium (Cell Science & Technology Institute) and cultured for 14 days. At that time, beads to which a recognition substance and a co-stimulating substance (EPHB4-CD80-4-1BBL) were bound were added on day 1 and day 8 after electroporation, and the cells were expansion cultured.

Separation using a specific factor other than CD45RA+ was performed in the same manner except that CD62L+, CD28+, IL-7R+, CD3+, CD27+, CD44+, CD44RO-, or CD57- was used for separation.

The CAR-T cells obtained after culturing were subjected to cell count measurement, FCM analysis, and anticancer tests. The results thereof are Table 5. In the Table, "-" shows a T cell population obtained without using a specific factor.

**[Table 5]**

| specific factor | before anticancer test | | | | | after anticancer test | | | | | |
|---|---|---|---|---|---|---|---|---|---|---|---|
| | number of CARpositive cells (relative value with "-" as 1.0) | T EMRA | T scm/ Naive | T EM | T CM | cancer cell survival rate (%) | increase/ decrease in CAR-positive rate | T EMRA | T scm/ Naïve | T EM | T CM |
| - | 1.0 | 35% | 30% | 19% | 17% | 0.2% | -53% | 17% | 17% | 30% | 36% |
| CD45RA+ | 7.2 | 54% | 40% | 2% | 1% | 0.0% | 16% | 17% | 17% | 23% | 43% |
| CD62L+ | 2.5 | 41% | 51% | 2% | 2% | 0.9% | 10% | 10% | 43% | 6% | 41% |
| CD28+ | 2.6 | 47% | 39% | 7% | 6% | 0.6% | -19% | 8% | 23% | 10% | 59% |
| IL-7R+ | 1.4 | 42% | 44% | 7% | 7% | 0.2% | -19% | 12% | 26% | 18% | 44% |
| CD3+ | 1.1 | 50% | 44% | 4% | 2% | 9.9% | -21% | 2% | 26% | 7% | 65% |
| CD27+ | 0.4 | 69% | 26% | 4% | 1% | 0.7% | -30% | 11% | 37% | 10% | 43% |
| CD44+ | 0.3 | 53% | 36% | 7% | 5% | 0.8% | -21% | 11% | 29% | 14% | 46% |
| CD44RO- | 0.2 | 54% | 30% | 9% | 7% | 0.6% | -37% | 15% | 24% | 32% | 37% |
| CD57- | 0.1 | 55% | 20% | 18% | 7% | 0.9% | -37% | 12% | 22% | 23% | 43% |

CAR-T cells prepared from a T cell subpopulation separated by specific factors have a high number of CAR-positive cells, and the results of FCM analysis show that the proportion of naive or young memory T cells (particularly, Tscm/naive and Tern) is also high, and PD-1 as an exhaustion marker was hardly expressed. Furthermore, anticancer effect was also high, and the presence of a sustained anticancer effect was suggested from the increase and decrease in the CAR-positive rate and the proportion of naive or young memory T cells after anticancer tests.

### Example 10

CAR-T cells were produced by activating CAR-T cells using a stimulating substance in the absence of feeder cells.

The CAR-T cells produced in Example 7 were cultured for 5-7 days in a culture medium supplemented with various stimulating substances. The stimulating substances used were: anti-CD62L antibody, PD0325901 (MEK inhibitor), SB431542 (TGF-βIR inhibitor), Rapamycin (mTOR inhibitor), simvastatin (HMG-CoA reductase inhibitor), GW9662, Rosiglitazone (PPARγ inhibitor), GW6471 (PPARα inhibitor), anti-CD327 antibody, anti-CD73 antibody, anti-CXCR5 antibody, anti-CCR7 antibody, anti-CCL5 antibody.

The CAR-T cells obtained after culturing were subjected to cell count measurement, FCM analysis, and anticancer tests. The results are shown in the following Table 6. Each value in the Table for items other than "cancer cell survival rate" "after anticancer test" is a relative value to that of Control.

**[Table 6]**

| stimulating substance | amount added | before anticancer test | | | | | after anticancer test | | | | | |
|---|---|---|---|---|---|---|---|---|---|---|---|---|
| | | CAR-positive rate | T EMRA | T scm/ Naive | T EM | T CM | cancer cell survival rate (%) | CAR-positive rate | T EMRA | T scm/ Naive | T EM | T CM |
| none (Control) | - | 1.0 | 1.0 | 1.0 | 1.0 | 1.0 | 0.99 | 1.0 | 1.0 | 1.0 | 1.0 | 1.0 |
| anti-CD62L antibody | 200 ng | 1.0 | 1.2 | 0.7 | 1.2 | 0.8 | 1.56 | 1.7 | 0.7 | 1.8 | 0.5 | 1.7 |
| PD0325901 | 10 nM | 0.9 | 0.9 | 1.1 | 1.1 | 1.2 | 0.95 | 0.6 | 0.2 | 1.2 | 0.3 | 1.2 |
| SB431542 | 10 *µ*M | 1.0 | 1.1 | 0.9 | 1.0 | 1.0 | 0.05 | 1.9 | 0.8 | 1.4 | 0.6 | 0.9 |
| | 10 nM | 0.9 | 1.0 | 1.0 | 1.1 | 1.2 | 0.06 | 1.2 | 0.2 | 1.5 | 0.2 | 1.0 |
| Rapamycin | 25 ng/mL | 1.0 | 1.2 | 0.6 | 1.8 | 1.0 | 0.11 | 1.5 | 0.2 | 1.5 | 0.3 | 1.0 |
| | 25 pg/mL | 1.0 | 0.9 | 1.2 | 0.9 | 1.2 | 0.54 | 1.3 | 0.5 | 1.4 | 0.3 | 1.0 |
| simvastatin | 10 nM | 1.0 | 1.1 | 0.8 | 1.4 | 1.0 | 0.28 | 1.2 | 0.5 | 1.1 | 0.4 | 1.1 |
| | 100 pM | 1.0 | 0.9 | 1.1 | 1.0 | 1.3 | 0.52 | 1.1 | 0.3 | 1.2 | 0.3 | 1.1 |
| GW9662 | 1 *µ*M | 1.0 | 1.3 | 0.7 | 1.3 | 0.7 | 0.70 | 1.2 | 0.7 | 1.3 | 0.8 | 1.4 |
| | 10 nM | 1.0 | 1.0 | 0.9 | 1.1 | 1.1 | 0.30 | 1.2 | 0.6 | 1.1 | 0.7 | 1.6 |
| Rosiglitazone | 10 *µ*M | 0.9 | 1.9 | 0.3 | 1.5 | 0.2 | 1.89 | 1.6 | 1.1 | 1.2 | 0.8 | 1.2 |
| | 1 *µ*M | 1.0 | 1.4 | 0.6 | 1.4 | 0.8 | 0.44 | 1.5 | 1.2 | 1.7 | 0.8 | 1.0 |
| GW6471 | 10 *µ*M | 1.0 | 1.7 | 0.5 | 1.4 | 0.3 | 2.22 | 1.8 | 1.3 | 1.4 | 1.0 | 0.8 |
| | 1 *µ*M | 1.0 | 1.5 | 0.5 | 1.4 | 0.4 | 1.11 | 1.5 | 1.4 | 1.7 | 0.9 | 0.9 |
| anti-CD327 antibody | 1 *µ*g | 1.0 | 1.0 | 1.0 | 1.0 | 0.9 | 0.36 | 1.1 | 1.3 | 1.2 | 0.9 | 1.0 |
| | 40 ng | 1.0 | 1.1 | 1.0 | 0.9 | 1.0 | 0.06 | 1.3 | 1.6 | 1.3 | 0.8 | 1.0 |
| anti-CD73 antibody | 1 *µ*g | 1.0 | 1.0 | 1.0 | 1.1 | 1.0 | 0.20 | 1.3 | 1.5 | 1.4 | 0.7 | 1.1 |
| | 40 ng | 1.0 | 0.9 | 1.0 | 1.0 | 1.1 | 0.08 | 1.4 | 1.6 | 1.3 | 0.9 | 1.0 |
| anti-CXCR5 antibody | 200 ng | 1.0 | 0.7 | 1.1 | 0.9 | 1.3 | 0.42 | 1.3 | 0.7 | 1.4 | 0.5 | 1.2 |
| | 40 ng | 1.0 | 1.1 | 0.9 | 1.2 | 0.7 | 0.76 | 1.1 | 0.7 | 1.2 | 0.7 | 1.1 |
| anti-CCR7 antibody | 5 *µ*g | 1.0 | 1.0 | 1.0 | 1.1 | 0.8 | 0.42 | 1.2 | 1.1 | 1.3 | 0.7 | 1.1 |
| | 40 ng | 1.0 | 1.3 | 0.9 | 1.2 | 0.7 | 0.18 | 1.1 | 1.1 | 1.2 | 1.0 | 0.9 |
| anti-CCL5 antibody | 5 *µ*g | 1.0 | 0.9 | 1.0 | 1.1 | 1.0 | 0.46 | 1.3 | 0.9 | 1.0 | 0.7 | 1.2 |
| | 200 ng | 1.0 | 0.9 | 1.0 | 1.1 | 0.9 | 0.50 | 1.0 | 0.9 | 1.0 | 0.8 | 1.1 |

As shown in Table 6, it was found that, when various stimulating substances are added, the proportion of younger and less exhausted T cells (particularly Tscm/naive and Tcm) increases even after the anticancer test as compared with the case without the addition of a stimulating substance (i.e., Control). At the same time, when a stimulating substance was added, the anticancer activity was the same as or increased as compared with the Control. The above results demonstrate that a high-quality CAR-T cell population that has a sustained and strong antitumor effect can be produced using specific stimulating substances.

### Example 11

A comparison was made between the conventional method and the present method regarding the production method of cell used for gene transfer and expansion culture method.

### 1. Production of EPHB4-CAR-T cells from PBMC

EPHB4-CAR-T cells were produced from cryopreserved PBMC. Frozen PBMC (CTL) was thawed by allowing to stand in a 37°C water bath, diluted with Dulbecco's phosphate buffered saline (D-PBS, Wako), and then the number of cells was measured using NC-3000 (M&S TechnoSystems, Inc.).

The thawed PBMCs were centrifuged, and the cell pellets were suspended in electroporation buffer (Miltenyi Biotec). PB transposase plasmid and pIRII-EPHB4-28z were added to the obtained cell suspension and introduced into PBMC by electroporation.

After gene transfer, the cells were transferred to a complete culture medium obtained by adding 5% artificial serum (Animal-free; Cell Science & Technology Institute), IL-7 (10 ng/mL; Miltenyi Biotec), and IL-15 (5 ng/mL; Miltenyi Biotec) to ALySTM705 Medium (Cell Science & Technology Institute) and cultured for 14 days. At that time, beads to which a recognition substance and a co-stimulating substance (EPHB4-CD80-4-1BBL) were bound were added on day 1 and day 8 after electroporation, and the cells were expansion cultured.

A sample with 25 ng/mL Rapamycin added and a sample with a feeder added at the same time as the addition of beads on day 1 was prepared simultaneously.

### Preparation of feeder cell

EPHB4-CAR-T cells were produced from cryopreserved PBMC. Frozen PBMC (CTL) were thawed by allowing to stand in a 37°C water bath, diluted with Dulbecco's phosphate buffered saline (D-PBS, Wako), and then the number of cells was measured using NC-3000 (M&S TechnoSystems, Inc.).

The thawed PBMCs were centrifuged, and the cell pellets were suspended in electroporation buffer (Miltenyi Biotec). PB transposase plasmid and pIRII-dEPHB4-CD80-BBL were added to the obtained cell suspension and introduced into PBMC by electroporation.

After gene transfer, the cells were transferred to a complete culture medium obtained by adding 5% artificial serum (Animal-free; Cell Science & Technology Institute), IL-7 (10 ng/mL; Miltenyi Biotec), and IL-15 (5 ng/mL; Miltenyi Biotec) to ALySTM705 Medium (Cell Science & Technology Institute) and cultured for 1 day. Thereafter, the cells were subjected to UV irradiation and equal amounts were added as a feeder to the cells into which CAR was introduced.

### 2. Production of EPHB4-CAR-T cells from cell population after

### separation with CD62L beads

Frozen PBMC (CTL) was thawed by allowing to stand in a 37°C water bath, diluted with Dulbecco's phosphate buffered saline (D-PBS, Wako), and then the number of cells was measured using NC-3000 (M&S TechnoSystems, Inc.).

The thawed PBMCs were centrifuged, and the cell pellets were suspended in MACS buffer (0.5% BSA, 2 mM EDTA in D-PBS), and cells expressing CD62L on the cell membrane surface (CD62L +) were separated using CD62L MicroBeads, human (Miltenyi Biotec, Bergisch Gladbach, Germany).

After separation, the number of cells was measured, the required amount of cell suspension was centrifuged, and the cell pellets were suspended in electroporation buffer. Thereafter, gene introduction and expansion culture were performed in the same manner as in the aforementioned "1."

The CAR-T cells obtained after culturing were subjected to cell count measurement, FCM analysis, and anticancer tests. The results are shown in the following Table 7. Each value in the Table for items other than "cancer cell survival rate" "after anticancer test" is a relative value to that of a T cell population produced using "co-stimulation beads" as a stimulation method in expansion culture of a cell population obtained with no "bead separation" ("-"). The co-stimulation beads used were MACSiBeads to which EPHB4, CD80, and 4-1BBL were bound.

**[Table 7]**

| bead separation | stimulation in expansion culture | before anticancer test | | | | | after anticancer test | | | | | |
|---|---|---|---|---|---|---|---|---|---|---|---|---|
| | | CAR-positive cell number | T EMRA | T scm/ Naïve | T EM | T CM | cancer cell survival rate (%) | CAR-positive rate | T EMRA | T scm/ Naïve | T EM | T CM |
| - | feeder cells | 0.1 | 1.2 | 1.4 | 0.5 | 0.6 | 0.3% | 2.4 | 1.2 | 4.2 | 0.4 | 1.4 |
| - | co-stimulation beads | 1.0 | 1.0 | 1.0 | 1.0 | 1.0 | 0.4% | 1.0 | 1.0 | 1.0 | 1.0 | 1.0 |
| - | co-stimulation beads + Rapamycin | 1.0 | 0.9 | 1.4 | 0.7 | 0.9 | 2.1% | 1.4 | 0.6 | 1.8 | 0.4 | 1.6 |
| CD62L | feeder cells | 0.1 | 0.3 | 2.7 | 0.2 | 0.4 | 0.5% | 2.3 | 0.9 | 8.9 | 0.2 | 1.2 |
| CD62L | co-stimulation beads | 4.2 | 1.0 | 1.9 | 0.3 | 0.4 | 1.8% | 1.8 | 0.7 | 5.7 | 0.2 | 1.5 |
| CD62L | co-stimulation beads + Rapamycin | 3.9 | 0.8 | 2.0 | 0.3 | 0.5 | 0.3% | 1.3 | 1.7 | 5.1 | 0.4 | 1.2 |

### Comparison before anticancer test

Comparing the feeder method and the feeder-free method, the feeder method had more Tscm/Naive cells but fewer CAR-positive cells. In the feeder-free method, even though Tscm/Naive decreased, about 30% or more was maintained under all conditions, and the number of CAR-positive cells was high. In CAR-T cells produced from PBMC using a feeder-free method, Tscm/Naive was higher with the additional addition of Rapamycin than with co-stimulation beads alone.

In addition, Tscm/Naive also increased in CAR-T cells produced after CD62L bead separation.

### Comparison after anticancer test

CAR-T cells that had a good Tscm/Naive ratio before the anticancer test tended to maintain the CAR-positive rate and Tscm/Naive ratio even after the anticancer test, suggesting a long-lasting sustainable anticancer effect.

### [Industrial Applicability]

According to the method of the present invention, CAR-T cells with higher quality can be obtained, and highly functional CAR-T cells can be produced conveniently ▪ in a short time ▪ at a low cost.

This application is based on a patent application No. 2021-179855 filed in Japan (filing date: November 2, 2021), the contents of which are incorporated in full herein.

## Claims

1. A method for producing a chimeric antigen receptor T (CAR-T) cell, comprising a step of separating the T cell from a T cell source with a specific factor, wherein
the specific factor is at least one kind selected from the group consisting of CD3+, CD4+, CD8+, CD45RA+, CD27+, IL-7R+, CCR7+, CD62L+, CD95+, CXCR5+, CD28+, CCL5+, CCL19+, CD45RB+, CD45RC+, CD80+, 4-1BBL+, CD44+, CD45RO-, CD57-, LAG3-, CXCR3-, and IL-2Rβ-, and
the separation step is at least one kind selected from the group consisting of a density gradient separation method, an immunological cell separation technique, a magnetic cell separation technique, a nylon wool separation method, and an adhesion method.

2. A method for producing a chimeric antigen receptor T (CAR-T) cell, comprising a step of bead separating a T cell from a T cell source with a specific factor, wherein
the specific factor is at least one kind selected from the group consisting of CD3+, CD4+, CD8+, CD45RA+, CD27+, IL-7R+, CCR7+, CD62L+, CD95+, CXCR5+, CD28+, CCL5+, CCL19+, CD45RB+, CD45RC+, CD80+, 4-1BBL+, CD44+, CD45RO-, CD57-, LAG3-, CXCR3-, and IL-2Rβ-.

3. The production method according to claim 1 or 2, wherein the specific factor is at least one kind selected from the group consisting of CD3+, CD45RA+, IL-7R+, CD62L+, and CD28+.

4. The production method according to claim 1 or 2, wherein the T cell source is a peripheral blood mononuclear cell.

5. A method for producing a chimeric antigen receptor T (CAR-T) cell, comprising a step of an activation treatment of a cell in the absence of a feeder cell.

6. The production method according to claim 5, wherein the cell is a T cell or a T cell that expresses a chimeric antigen receptor.

7. The production method according to claim 5 or 6, wherein the activation treatment is performed by contacting the cell with a stimulating substance.

8. The production method according to claim 7, wherein the contact with the stimulating substance is contact with a substrate to which the stimulating substance is bound.

9. The production method according to claim 8, wherein the contact with the substrate is co-culture of the cell and the substrate.

10. The production method according to claim 5 or 6, wherein the activation treatment is performed by culturing the cell in a medium containing a stimulating substance.

11. The production method according to claim 10, wherein the activation treatment is performed by co-culturing the cell and a substrate, to which a stimulating substance is bound, in a medium containing a stimulating substance.

12. The production method according to claim 11, wherein the substrate to which a stimulating substance is bound is a bead to which a protein is bound.

13. The production method according to claim 7, wherein the stimulating substance is at least one kind selected from the group consisting of CD4+, CD8+, CD45RA+, CCR7+, CD62L+, CXCR5+, CCL5+, CD327+, CD73+, CCL19+, CD45RB+, CD3-, CD45RO-, CD57-, LAG3-, CXCR3-, IL-2Rβ-, BCL2L14-, MEK1-, MEK2-, mTOR-, PPAR-, and statin-.

14. The production method according to claim 13, wherein the stimulating substance is at least one kind selected from the group consisting of CCR7+, CD62L+, CXCR5+, CCL5+, CD327+, CD73+, SB431542-, PD0325901-, Rapamycin-, simvastatin-, GW9662-, Rosiglitazone-, and GW6471-.

15. A method for producing a chimeric antigen receptor T (CAR-T) cell, comprising a step of an antigen recognition treatment of a cell in the absence of a feeder cell.

16. The production method according to claim 15, wherein the cell is a T cell or a T cell that expresses a chimeric antigen receptor.

17. The production method according to claim 15 or 16,
wherein the antigen recognition treatment is performed by contacting the cell with a recognition substance.

18. The production method according to claim 17, wherein the contact with the recognition factor is contact with a substrate to which a recognition substance is bound.

19. The production method according to claim 18, wherein the contact with the substrate is co-culture of the cell and the substrate.

20. The production method according to claim 15 or 16,
wherein the antigen recognition treatment is performed by culturing the cell in a medium containing a recognition substance.

21. The production method according to claim 20, wherein the antigen recognition treatment is performed by co-culturing the cell and a substrate, to which a recognition substance is bound, in a medium containing a recognition substance.

22. The production method according to claim 21, wherein the substrate to which the recognition substance is bound is a bead to which a protein of the recognition substance is bound.

23. The production method according to claim 17, wherein the recognition substance is at least one kind selected from the group consisting of EPHB4, HER2, and CD19.

24. The production method according to claim 5 or 15, wherein a cell after CAR gene introduction and a bead, to which a stimulating substance and a recognition substance are bound, are co-cultured in a medium containing a stimulating substance and a recognition substance, wherein
the stimulating substance is at least one kind selected from the group consisting of CCR7+, CD62L+, CXCR5+, CCL5+, CD327+, CD73+, SB431542-, PD0325901-, Rapamycin-, simvastatin-, GW9662-, Rosiglitazone-, and GW6471-, and
the recognition substance is at least one kind selected from the group consisting of EPHB4, HER2, and CD19.

25. A method for producing a chimeric antigen receptor T (CAR-T) cell, comprising
a step of separating a T cell from a T cell source with a specific factor,
a step of introducing a CAR gene into the separated cell, and
a step of expansion culture of the cell into which the CAR gene has been introduced, wherein
the specific factor is at least one kind selected from the group consisting of CD3+, CD45RA+, CD27+, IL-7R+, CD62L+, CD28+, CD44+, CD45RO-, and CD57-.

26. The production method according to claim 25, wherein the separation step is bead separation.

27. The production method according to claim 25 or 26, wherein the specific factor is at least one kind selected from the group consisting of CD3+, CD45RA+, IL-7R+, CD62L+, and CD28+.

28. The production method according to claim 25, wherein the expansion culture comprises an activation treatment and an antigen recognition treatment.

29. The production method according to claim 28, wherein the activation treatment is performed by contacting the cell with a stimulating substance.

30. The production method according to claim 29, wherein the contact with the stimulating substance is contact with a substrate to which the stimulating substance is bound.

31. The production method according to claim 30, wherein the contact with the substrate is co-culture of the cell and the substrate.

32. The production method according to claim 28 or 29, wherein the activation treatment is performed by culturing the cell in a medium containing a stimulating substance.

33. The production method according to claim 32, wherein the activation treatment is performed by co-culturing the cell and a substrate, to which a stimulating substance is bound, in a medium containing a stimulating substance.

34. The production method according to claim 33, wherein the substrate to which a stimulating substance is bound is a bead to which a protein is bound.

35. The production method according to claim 29, wherein the stimulating substance is at least one kind selected from the group consisting of CCR7+, CD62L+, CXCR5+, CCL5+, CD327+, CD73+, SB431542-, PD0325901-, Rapamycin-, simvastatin-, GW9662-, Rosiglitazone-, and GW6471-.

36. The production method according to claim 28, wherein the antigen recognition treatment is performed by contacting the cell with a recognition substance.

37. The production method according to claim 36, wherein the contact with the recognition substance is contact with a substrate to which a recognition substance is bound.

38. The production method according to claim 37, wherein the contact with the substrate is co-culture of the cell and the substrate.

39. The production method according to claim 28 or 36, wherein the antigen recognition treatment is performed by culturing the cell in a medium containing a recognition substance.

40. The production method according to claim 39, wherein the antigen recognition treatment is performed by co-culturing the cell and a substrate, to which a recognition substance is bound, in a medium containing a recognition substance.

41. The production method according to claim 40, wherein the substrate to which a recognition substance is bound is a bead to which a protein is bound.

42. The production method according to claim 39, wherein the recognition substance is at least one kind selected from the group consisting of EPHB4, HER2, and CD19.

43. The production method according to claim 28, wherein a cell after CAR gene introduction and a bead, to which a stimulating substance and a recognition substance are bound, are co-cultured in a medium containing a stimulating substance and a recognition substance, wherein
the stimulating substance is at least one kind selected from the group consisting of CCR7+, CD62L+, CXCR5+, CCL5+, CD327+, CD73+, SB431542-, PD0325901-, Rapamycin-, simvastatin-, GW9662-, Rosiglitazone-, and GW6471-, and
the recognition substance is at least one kind selected from the group consisting of EPHB4, HER2, and CD19.

44. A method for producing a chimeric antigen receptor T (CAR-T) cell, comprising co-culturing, in the absence of a feeder cell and in a medium containing a stimulating substance, a cell after CAR gene introduction and a bead, to which a co-stimulating substance and a recognition substance are bound, wherein
the stimulating substance is at least one kind selected from the group consisting of an anti-CCR7 antibody, an anti-CD62L antibody, an anti-CXCR5 antibody, an anti-CCL5 antibody, an anti-CD327 antibody, an anti-CD73 antibody, SB431542, PD0325901, Rapamycin, simvastatin, GW9662, Rosiglitazone, and GW6471,
the recognition substance is at least one kind selected from the group consisting of EPHB4, HER2, and CD19,
the co-stimulating substance is at least one kind selected from the group consisting of CD80, CD86, 4-1BBL, OX40L, ICOS-L, CD70, CD40L, CD270, ICAM-1, LFA-3, CD72, CD55, VCAM-1, MadCAM-1, CD111, CD112, CD155, CD153, PD-L2, PD-L1, Galectin-9, MHC, and CD113.

45. The production method according to claim 44, wherein the cell after CAR gene introduction is a cell derived from a T cell separated with a specific factor from a T cell source, and
the specific factor is at least one kind selected from the group consisting of CD3+, CD45RA+, IL-7R+, CD62L+, and CD28+.
